(19) Europäisches Patentamt
European Patent Office
Office européen des brevets

(11) EP 3 118 315 A1

(12) **EUROPEAN PATENT APPLICATION**
published in accordance with Art. 153(4) EPC

(43) Date of publication:
**18.01.2017 Bulletin 2017/03**

(21) Application number: **15761287.0**

(22) Date of filing: **12.03.2015**

(51) Int Cl.:
***C12N 15/113*** *(2010.01)*

(86) International application number:
**PCT/JP2015/057353**

(87) International publication number:
**WO 2015/137459 (17.09.2015 Gazette 2015/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **13.03.2014 US 201461952426 P**

(71) Applicant: **Kyowa Hakko Kirin Co., Ltd.**
**Tokyo 100-8185 (JP)**

(72) Inventors:
- **CHEN, Lishan**
  **La Jolla, California 92037 (US)**
- **MILLS, David**
  **La Jolla, California 92037 (US)**
- **GROFF, Brian**
  **San Diego, California 92127 (US)**

(74) Representative: **Vossius & Partner**
**Patentanwälte Rechtsanwälte mbB**
**Siebertstrasse 3**
**81675 München (DE)**

# (54) NUCLEIC ACID THAT INHIBITS EXPRESSION OF IRF5

(57) The present invention provides a nucleic acid having activity to suppress expression of IRF5, a pharmaceutical composition comprising the nucleic acid, and a prophylactic or therapeutic drug containing the nucleic acid for autoimmune diseases such as systemic lupus erythematosus, rheumatoid arthritis and the like.

EP 3 118 315 A1

SEQUENCE LISTING

<110>  KYOWA HAKKO KIRIN CO., LTD

<120>  Nucleic Acids for Inhibiting Expression of IRF5

<130>  092302

<150>  US 61/952,426
<151>  2014-03-13

<160>  238

<170>  PatentIn version 3.5

<210>  1
<211>  25
<212>  DNA
<213>  Homo sapiens

<400>  1
ccagtccatc ccagtggctc ccacc    25

<210>  2
<211>  25
<212>  DNA
<213>  Homo sapiens

<400>  2
ggctggtggc ccaggtgaac agctg    25

<210>  3
<211>  25
<212>  DNA
<213>  Homo sapiens

<400>  3
caggtgaaca gctgccagta cccag    25

<210>  4
<211>  25
<212>  DNA
<213>  Homo sapiens

<400>  4
gggtcaacgg ggaaaagaaa ttatt    25

<210>  5
<211>  25
<212>  DNA
<213>  Homo sapiens

<400>  5
ggtcaacggg gaaaagaaat tattc    25

<210>  6
<211>  25
<212>  DNA

<213> Homo sapiens

<400> 6
acggggaaaa gaaattattc tgcat 25

<210> 7
<211> 25
<212> DNA
<213> Homo sapiens

<400> 7
gtcccagcca ggacggagat aacac 25

<210> 8
<211> 25
<212> DNA
<213> Homo sapiens

<400> 8
gagataacac catcttcaag gcctg 25

<210> 9
<211> 25
<212> DNA
<213> Homo sapiens

<400> 9
gggccaagga gacagggaaa tacac 25

<210> 10
<211> 25
<212> DNA
<213> Homo sapiens

<400> 10
ggccaaggag acagggaaat acacc 25

<210> 11
<211> 25
<212> DNA
<213> Homo sapiens

<400> 11
caccgaaggc gtggatgaag ccgat 25

<210> 12
<211> 25
<212> DNA
<213> Homo sapiens

<400> 12
ccgatccggc caagtggaag gccaa 25

<210> 13
<211> 25
<212> DNA

<213> Homo sapiens

<400> 13
ccggccaagt ggaaggccaa cctgc 25

<210> 14
<211> 25
<212> DNA
<213> Homo sapiens

<400> 14
cggccaagtg gaaggccaac ctgcg 25

<210> 15
<211> 25
<212> DNA
<213> Homo sapiens

<400> 15
tgcgctgtgc ccttaacaag agccg 25

<210> 16
<211> 25
<212> DNA
<213> Homo sapiens

<400> 16
gggacttccg cctcatctac gacgg 25

<210> 17
<211> 25
<212> DNA
<213> Homo sapiens

<400> 17
caatggccct gctcccacag actcc 25

<210> 18
<211> 25
<212> DNA
<213> Homo sapiens

<400> 18
ccctgaggat tactcttttg gtgca 25

<210> 19
<211> 25
<212> DNA
<213> Homo sapiens

<400> 19
ggattactct tttggtgcag gagag 25

<210> 20
<211> 25
<212> DNA

<213> Homo sapiens

<400> 20
gagaggagga ggaagaagag gaaga 25

<210> 21
<211> 25
<212> DNA
<213> Homo sapiens

<400> 21
ggaggaggaa gaagaggaag agctg 25

<210> 22
<211> 25
<212> DNA
<213> Homo sapiens

<400> 22
gcctgagcct cacagaggat gtcaa 25

<210> 23
<211> 25
<212> DNA
<213> Homo sapiens

<400> 23
gcctcacaga ggatgtcaag tggcc 25

<210> 24
<211> 25
<212> DNA
<213> Homo sapiens

<400> 24
ctggcttcag ggagcttctc tctga 25

<210> 25
<211> 25
<212> DNA
<213> Homo sapiens

<400> 25
gcgaacagct cctgccagac ctgct 25

<210> 26
<211> 25
<212> DNA
<213> Homo sapiens

<400> 26
ctctgaccga cctggagatc aagtt 25

<210> 27
<211> 25
<212> DNA

<213> Homo sapiens

<400> 27
tgaccgacct ggagatcaag tttca 25

<210> 28
<211> 25
<212> DNA
<213> Homo sapiens

<400> 28
cgacctggag atcaagtttc agtac 25

<210> 29
<211> 25
<212> DNA
<213> Homo sapiens

<400> 29
acctggagat caagtttcag taccg 25

<210> 30
<211> 25
<212> DNA
<213> Homo sapiens

<400> 30
gggccctcac catcagcaac cccca 25

<210> 31
<211> 25
<212> DNA
<213> Homo sapiens

<400> 31
cccaggagca ggtggaactc ttcgg 25

<210> 32
<211> 25
<212> DNA
<213> Homo sapiens

<400> 32
ggaactcttc ggccccataa gcctg 25

<210> 33
<211> 25
<212> DNA
<213> Homo sapiens

<400> 33
agcagcgctt ctacacgaac cagct 25

<210> 34
<211> 25
<212> DNA

<213> Homo sapiens

<400> 34
acacgaacca gctgctggat gtcct 25

<210> 35
<211> 25
<212> DNA
<213> Homo sapiens

<400> 35
cgaaccagct gctggatgtc ctgga 25

<210> 36
<211> 25
<212> DNA
<213> Homo sapiens

<400> 36
cgggctcatc ctccagctac agggc 25

<210> 37
<211> 25
<212> DNA
<213> Homo sapiens

<400> 37
ggctcatcct ccagctacag ggcca 25

<210> 38
<211> 25
<212> DNA
<213> Homo sapiens

<400> 38
ctgtgtcagt gcaaggtgtt ctgga 25

<210> 39
<211> 25
<212> DNA
<213> Homo sapiens

<400> 39
ccatccagcg ggaggtcaag accaa 25

<210> 40
<211> 25
<212> DNA
<213> Homo sapiens

<400> 40
agcgggaggt caagaccaag ctttt 25

<210> 41
<211> 25
<212> DNA

<213> Homo sapiens

<400> 41
ggaggtcaag accaagcttt tcagc 25

<210> 42
<211> 25
<212> DNA
<213> Homo sapiens

<400> 42
gcctggagca ttttctcaat gagct 25

<210> 43
<211> 25
<212> DNA
<213> Homo sapiens

<400> 43
tcaatgagct catcctgttc caaaa 25

<210> 44
<211> 25
<212> DNA
<213> Homo sapiens

<400> 44
agctcatcct gttccaaaag ggcca 25

<210> 45
<211> 25
<212> DNA
<213> Homo sapiens

<400> 45
caccottcga gatcttcttc tgctt 25

<210> 46
<211> 25
<212> DNA
<213> Homo sapiens

<400> 46
gcaaaccccg agagaagaag ctcat 25

<210> 47
<211> 25
<212> DNA
<213> Homo sapiens

<400> 47
cgagagaaga agctcattac tgtac 25

<210> 48
<211> 25
<212> DNA

<213> Homo sapiens

<400> 48
gaagaagctc attactgtac aggtg 25

<210> 49
<211> 25
<212> DNA
<213> Homo sapiens

<400> 49
cagctcgact gctgctggag atgtt 25

<210> 50
<211> 25
<212> DNA
<213> Homo sapiens

<400> 50
gagctatctt ggtcagctga tagta 25

<210> 51
<211> 25
<212> DNA
<213> Homo sapiens

<400> 51
ctatcttggt cagctgatag tatcc 25

<210> 52
<211> 25
<212> DNA
<213> Homo sapiens

<400> 52
agctgatagt atccggctac agatc 25

<210> 53
<211> 25
<212> DNA
<213> Homo sapiens

<400> 53
ctgatagtat ccggctacag atctc 25

<210> 54
<211> 25
<212> DNA
<213> Homo sapiens

<400> 54
gatagtatcc ggctacagat ctcaa 25

<210> 55
<211> 25
<212> DNA

<213> Homo sapiens

<400> 55
gctacagatc tcaaacccag acctc 25

<210> 56
<211> 25
<212> DNA
<213> Homo sapiens

<400> 56
cgcatggtgg agcaattcaa ggagc 25

<210> 57
<211> 25
<212> DNA
<213> Homo sapiens

<400> 57
gcatggtgga gcaattcaag gagct 25

<210> 58
<211> 25
<212> DNA
<213> Homo sapiens

<400> 58
caaggagctc catcacatct ggcag 25

<210> 59
<211> 25
<212> DNA
<213> Homo sapiens

<400> 59
gcacccagct ggcatgcaat aacaa 25

<210> 60
<211> 25
<212> DNA
<213> Homo sapiens

<400> 60
cccagctggc atgcaataac aaggc 25

<210> 61
<211> 25
<212> DNA
<213> Homo sapiens

<400> 61
cagctggcat gcaataacaa ggctg 25

<210> 62
<211> 25
<212> DNA

<213> Homo sapiens

<400> 62
agctggcatg caataacaag gctgc 25

<210> 63
<211> 25
<212> DNA
<213> Homo sapiens

<400> 63
gactgatgtg gagatgtgac agccc 25

<210> 64
<211> 25
<212> DNA
<213> Homo sapiens

<400> 64
cagggtccta cctctgggtt tcctg 25

<210> 65
<211> 25
<212> DNA
<213> Homo sapiens

<400> 65
gggtttcctg gaagtggatt tgggc 25

<210> 66
<211> 25
<212> DNA
<213> Homo sapiens

<400> 66
ggaagtggat ttgggccaag aagga 25

<210> 67
<211> 25
<212> DNA
<213> Homo sapiens

<400> 67
ccatgagcag ggaaagaact ctccc 25

<210> 68
<211> 25
<212> DNA
<213> Homo sapiens

<400> 68
cctggggcct agctgtatag gagga 25

<210> 69
<211> 25
<212> DNA

<213> Homo sapiens

<400> 69
catttcctct ggcaacaaaa gccag 25

<210> 70
<211> 25
<212> DNA
<213> Homo sapiens

<400> 70
ctcacttcct catctccctg tcctc 25

<210> 71
<211> 25
<212> DNA
<213> Homo sapiens

<400> 71
ccctgtcctc tgagataata tgagt 25

<210> 72
<211> 25
<212> DNA
<213> Homo sapiens

<400> 72
gcacttaggt atcatatcag atgct 25

<210> 73
<211> 25
<212> DNA
<213> Homo sapiens

<400> 73
atatcagatg ctcaaggctg gcagc 25

<210> 74
<211> 25
<212> DNA
<213> Homo sapiens

<400> 74
ccccttcttg agagtccaag aacct 25

<210> 75
<211> 25
<212> DNA
<213> Homo sapiens

<400> 75
ccttcttgag agtccaagaa cctgg 25

<210> 76
<211> 25
<212> DNA

<213> Homo sapiens

<400> 76
ccaagaacct ggagcagaaa taatt 25

<210> 77
<211> 25
<212> DNA
<213> Homo sapiens

<400> 77
gaacctggag cagaaataat tttta 25

<210> 78
<211> 25
<212> DNA
<213> Homo sapiens

<400> 78
ggagcagaaa taatttttat gtatt 25

<210> 79
<211> 25
<212> DNA
<213> Homo sapiens

<400> 79
gattaatgaa tgttaaaaac agact 25

<210> 80
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxycytidine

<400> 80
ccaguccauc ccaguggcuc ccann 25

<210> 81
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxyguanosine

<400> 81
ggcugguggc ccaggugaac agcnn 25

<210> 82
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxyguanosine

<400> 82
caggugaaca gcugccagua cccnn 25

<210> 83
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxythymidine

<400> 83
gggucaacgg ggaaaagaaa uuann 25

<210> 84

<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxycytidine

<400> 84
ggucaacggg gaaaagaaau uaunn 25

<210> 85
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxythymidine

<400> 85
acggggaaaa gaaauuauuc ugcnn 25

<210> 86
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxycytidine

<400> 86
gucccagcca ggacggagau aacnn 25

<210> 87
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxyguanosine

<400> 87
gagauaacac caucuucaag gccnn 25

<210> 88
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxycytidine

<400> 88
gggccaagga gacagggaaa uacnn 25

<210> 89
<211> 25
<212> RNA
<213> Artificial Sequence

<220>
<223> Combined DNA/RNA Molecule: Synthetic oligonucleotide

<220>
<221> misc_feature
<222> (24)..(24)

<223>  deoxycytidine

<220>
<221>  misc_feature
<222>  (25)..(25)
<223>  deoxycytidine

<400>  89
ggccaaggag acagggaaau acann                                           25

<210>  90
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxyadenosine

<220>
<221>  misc_feature
<222>  (25)..(25)
<223>  deoxythymidine

<400>  90
caccgaaggc guggaugaag ccgnn                                           25

<210>  91
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxyadenosine

<220>
<221>  misc_feature
<222>  (25)..(25)
<223>  deoxyadenosine

<400>  91
ccgauccggc caaguggaag gccnn                                           25

<210>  92
<211>  25
<212>  RNA
<213>  Artificial Sequence

<220>

<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxycytidine

<400> 92
ccggccaagu ggaaggccaa ccunn 25

<210> 93
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxyguanosine

<400> 93
cggccaagug gaaggccaac cugnn 25

<210> 94
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxyguanosine

<400> 94
ugcgcugugc ccuuaacaag agcnn 25

<210> 95
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxyguanosine

<400> 95
gggacuuccg ccucaucuac gacnn 25

<210> 96
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxycytidine

<400> 96
caauggcccu gcucccacag acunn 25

<210> 97
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)

<223>  deoxyadenosine

<400>  97
cccugaggau uacucuuuug gugnn 25

<210>  98
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxyadenosine

<220>
<221>  misc_feature
<222>  (25)..(25)
<223>  deoxyguanosine

<400>  98
ggauuacucu uuuggugcag gagnn 25

<210>  99
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxyguanosine

<220>
<221>  misc_feature
<222>  (25)..(25)
<223>  deoxyadenosine

<400>  99
gagaggagga ggaagaagag gaann 25

<210>  100
<211>  25
<212>  RNA
<213>  Artificial Sequence

<220>
<223>  Combined DNA/RNA Molecule: Synthetic oligonucleotide

<220>
<221>  misc_feature

<222>  (24)..(24)
<223>  deoxythymidine

<220>
<221>  misc_feature
<222>  (25)..(25)
<223>  deoxyguanosine

<400>  100
ggaggaggaa gaagaggaag agcnn                                           25

<210>  101
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxyadenosine

<220>
<221>  misc_feature
<222>  (25)..(25)
<223>  deoxyadenosine

<400>  101
gccugagccu cacagaggau gucnn                                           25

<210>  102
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxycytidine

<220>
<221>  misc_feature
<222>  (25)..(25)
<223>  deoxycytidine

<400>  102
gccucacaga ggaugucaag uggnn                                           25

<210>  103
<211>  25
<212>  RNA
<213>  Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxyadenosine

<400> 103
cuggcuucag ggagcuucuc ucunn 25

<210> 104
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxythymidine

<400> 104
gcgaacagcu ccugccagac cugnn 25

<210> 105
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxythymidine

<400> 105
cucugaccga ccuggagauc aagnn 25

<210>  106
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxycytidine

<220>
<221>  misc_feature
<222>  (25)..(25)
<223>  deoxyadenosine

<400>  106
ugaccgaccu ggagaucaag uuunn    25

<210>  107
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxyadenosine

<220>
<221>  misc_feature
<222>  (25)..(25)
<223>  deoxycytidine

<400>  107
cgaccuggag aucaaguuuc agunn    25

<210>  108
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxycytidine

<220>
<221>  misc_feature

<222>  (25)..(25)
<223>  deoxyguanosine

<400>  108
accuggagau caaguuucag uacnn                                            25

<210>  109
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxycytidine

<220>
<221>  misc_feature
<222>  (25)..(25)
<223>  deoxyadenosine

<400>  109
gggcccucac caucagcaac cccnn                                            25

<210>  110
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxyguanosine

<220>
<221>  misc_feature
<222>  (25)..(25)
<223>  deoxyguanosine

<400>  110
cccaggagca gguggaacuc uucnn                                            25

<210>  111
<211>  25
<212>  RNA
<213>  Artificial Sequence

<220>
<223>  Combined DNA/RNA Molecule: Synthetic oligonucleotide

<220>

<221> misc_feature
<222> (24)..(24)
<223> deoxythymidine

<220>
<221> misc_feature
<222> (25)..(25)
<223> deoxyguanosine

<400> 111
ggaacucuuc ggccccauaa gccnn 25

<210> 112
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxythymidine

<400> 112
agcagcgcuu cuacacgaac cagnn 25

<210> 113
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxythymidine

<400> 113
acacgaacca gcugcuggau gucnn 25

<210> 114
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxyadenosine

<400> 114
cgaaccagcu gcuggauguc cugnn 25

<210> 115
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxycytidine

<400> 115
cgggcucauc cuccagcuac aggnn 25

<210> 116
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxyadenosine

<400> 116
ggcucauccu ccagcuacag ggcnn 25

<210> 117
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxyadenosine

<400> 117
cugugucagu gcaagguguu cugnn 25

<210> 118
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxyadenosine

<400> 118
ccauccagcg ggaggucaag accnn 25

<210> 119
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>

<221> misc_feature
<222> (25)..(25)
<223> deoxythymidine

<400> 119
agcgggaggu caagaccaag cuunn 25

<210> 120
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxycytidine

<400> 120
ggaggucaag accaagcuuu ucann 25

<210> 121
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxythymidine

<400> 121
gccuggagca uuuucucaau gagnn 25

<210> 122
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxyadenosine

<400> 122
ucaaugagcu cauccuguuc caann 25

<210> 123
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxyadenosine

<400> 123
agcucauccu guuccaaaag ggcnn 25

<210> 124
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxythymidine

<400> 124
cacccuucga gaucuucuuc ugcnn 25

<210> 125
<211> 25
<212> RNA

<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxythymidine

<400> 125
gcaaaccccg agagaagaag cucnn 25

<210> 126
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxycytidine

<400> 126
cgagagaaga agcucauuac ugunn 25

<210> 127
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxyguanosine

<400> 127

gaagaagcuc auuacuguac aggnn 25

<210> 128
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxythymidine

<400> 128
cagcucgacu gcugcuggag augnn 25

<210> 129
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxyadenosine

<400> 129
gagcuaucuu ggucagcuga uagnn 25

<210> 130
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxycytidine

<400> 130
cuaucuuggu cagcugauag uaunn 25

<210> 131
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxycytidine

<400> 131
agcugauagu auccggcuac agann 25

<210> 132
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxycytidine

<400> 132
cugauaguau ccggcuacag aucnn 25

<210> 133
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxyadenosine

<400> 133
gauaguaucc ggcuacagau cucnn 25

<210> 134
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxycytidine

<400> 134
gcuacagauc ucaaacccag accnn 25

<210> 135
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxycytidine

<400> 135
cgcauggugg agcaauucaa ggann 25

<210> 136
<211> 25

<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxythymidine

<400> 136
gcauggugga gcaauucaag gagnn 25

<210> 137
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxyguanosine

<400> 137
caaggagcuc caucacaucu ggcnn 25

<210> 138
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxyadenosine

<400> 138
gcacccagcu ggcaugcaau aacnn 25

<210> 139
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxycytidine

<400> 139
cccagcuggc augcaauaac aagnn 25

<210> 140
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxyguanosine

<400> 140
cagcuggcau gcaauaacaa ggcnn 25

<210> 141
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxycytidine

<400> 141
agcuggcaug caauaacaag gcunn 25

<210> 142
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxycytidine

<400> 142
gacugaugug gagaugugac agcnn 25

<210> 143
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxyguanosine

<400> 143
cagggguccua ccucuggguu uccnn 25

<210> 144
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxycytidine

<400> 144
ggguuuccug gaaguggauu uggnn 25

<210> 145
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxyadenosine

<400> 145
ggaaguggau uugggccaag aagnn 25

<210> 146
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxycytidine

<400> 146
ccaugagcag ggaaagaacu cucnn 25

<210> 147

<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxyadenosine

<400> 147
ccuggggccu agcuguauag gagnn 25

<210> 148
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyadenosine

<220>
<221> misc_feature
<222> (25)..(25)
<223> deoxyguanosine

<400> 148
cauuuccucu ggcaacaaaa gccnn 25

<210> 149
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxythymidine

<220>
<221> misc_feature
<222> (25)..(25)
<223> deoxycytidine

<400> 149
cucacuuccu caucucccug uccnn 25

<210> 150
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxythymidine

<400> 150
cccuguccuc ugagauaaua ugann 25

<210> 151
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxythymidine

<400> 151
gcacuuaggu aucauaucag augnn 25

<210> 152
<211> 25
<212> RNA
<213> Artificial Sequence

<220>
<223> Combined DNA/RNA Molecule: Synthetic oligonucleotide

<220>
<221> misc_feature
<222> (24)..(24)

<223> deoxyguanosine

<220>
<221> misc_feature
<222> (25)..(25)
<223> deoxycytidine

<400> 152
auaucagaug cucaaggcug gcann 25

<210> 153
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxythymidine

<400> 153
ccccuucuug agaguccaag aacnn 25

<210> 154
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxyguanosine

<220>
<221> misc_feature
<222> (25)..(25)
<223> deoxyguanosine

<400> 154
ccuucuugag aguccaagaa ccunn 25

<210> 155
<211> 25
<212> RNA
<213> Artificial Sequence

<220>

<223>  Combined DNA/RNA Molecule: Synthetic oligonucleotide

<220>
<221>  misc_feature
<222>  (24)..(24)
<223>  deoxythymidine

<220>
<221>  misc_feature
<222>  (25)..(25)
<223>  deoxythymidine

<400>  155
ccaagaaccu ggagcagaaa uaann                                                25

<210>  156
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxythymidine

<220>
<221>  misc_feature
<222>  (25)..(25)
<223>  deoxyadenosine

<400>  156
gaaccuggag cagaaauaau uuunn                                                25

<210>  157
<211>  25
<212>  RNA
<213>  Artificial Sequence

<220>
<223>  Combined DNA/RNA Molecule: Synthetic oligonucleotide

<220>
<221>  misc_feature
<222>  (24)..(24)
<223>  deoxythymidine

<220>
<221>  misc_feature
<222>  (25)..(25)
<223>  deoxythymidine

<400>  157
ggagcagaaa uaauuuuuau guann                                                25

<210> 158
<211> 25
<212> RNA
<213> Artificial Sequence

<220>
<223> Combined DNA/RNA Molecule: Synthetic oligonucleotide

<220>
<221> misc_feature
<222> (24)..(24)
<223> deoxycytidine

<220>
<221> misc_feature
<222> (25)..(25)
<223> deoxythymidine

<400> 158
gauuaaugaa uguuaaaaac agann 25

<210> 159
<211> 27
<212> RNA
<213> Artificial Sequence

<220>
<223> Synthetic oligonucleotide

<400> 159
ggugggagcc acugggaugg acugguu 27

<210> 160
<211> 27
<212> RNA
<213> Artificial Sequence

<220>
<223> Synthetic oligonucleotide

<400> 160
cagcuguuca ccugggccac cagccag 27

<210> 161
<211> 27
<212> RNA
<213> Artificial Sequence

<220>
<223> Synthetic oligonucleotide

<400> 161
cuggguacug gcagcuguuc accuggg 27

<210> 162
<211> 27
<212> RNA
<213> Artificial Sequence

<220>
<223> Synthetic oligonucleotide

<400> 162
aauaauuucu uuuccccguu gacccau 27

<210> 163
<211> 27
<212> RNA
<213> Artificial Sequence

<220>
<223> Synthetic oligonucleotide

<400> 163
gaauaauuuc uuuuccccgu ugaccca 27

<210> 164
<211> 27
<212> RNA
<213> Artificial Sequence

<220>
<223> Synthetic oligonucleotide

<400> 164
augcagaaua auuucuuuuc cccguug 27

<210> 165
<211> 27
<212> RNA
<213> Artificial Sequence

<220>
<223> Synthetic oligonucleotide

<400> 165
guguuaucuc cguccuggcu gggacca 27

<210> 166
<211> 27
<212> RNA
<213> Artificial Sequence

<220>
<223> Synthetic oligonucleotide

<400> 166
caggccuuga agauggugau aucuccg 27

<210> 167
<211> 27
<212> RNA
<213> Artificial Sequence

<220>
<223> Synthetic oligonucleotide

<400> 167
guguauuucc cugucuccuu ggcccag 27

<210> 168
<211> 27
<212> RNA
<213> Artificial Sequence

<220>
<223> Synthetic oligonucleotide

<400> 168
ggugguguuuc ccugucuccu uggccca 27

<210> 169
<211> 27
<212> RNA
<213> Artificial Sequence

<220>
<223> Synthetic oligonucleotide

<400> 169
aucggcuuca uccacgccuu cggugua 27

<210> 170
<211> 27
<212> RNA
<213> Artificial Sequence

<220>
<223> Synthetic oligonucleotide

<400> 170
uuggccuucc acuuggccgg aucggcu 27

<210> 171
<211> 27
<212> RNA
<213> Artificial Sequence

<220>
<223> Synthetic oligonucleotide

<400> 171
gcagguuggc cuuccacuug gccggau 27

<210> 172
<211> 27
<212> RNA
<213> Artificial Sequence

<220>
<223> Synthetic oligonucleotide

<400> 172
cgcagguugg ccuuccacuu ggccgga 27

<210> 173
<211> 27
<212> RNA
<213> Artificial Sequence

<220>
<223> Synthetic oligonucleotide

<400> 173
cggcucuugu uaagggcaca gcgcagg 27

<210> 174
<211> 27
<212> RNA
<213> Artificial Sequence

<220>
<223> Synthetic oligonucleotide

<400> 174
ccgucguaga ugaggcggaa gucccgg 27

<210> 175
<211> 27
<212> RNA
<213> Artificial Sequence

<220>
<223> Synthetic oligonucleotide

<400> 175
ggagucugug ggagcagggc cauugga 27

<210> 176
<211> 27
<212> RNA
<213> Artificial Sequence

<220>
<223> Synthetic oligonucleotide

<400> 176
ugcaccaaaa gaguaauccu caggggg 27

<210> 177
<211> 27
<212> RNA
<213> Artificial Sequence

<220>
<223> Synthetic oligonucleotide

<400> 177
cucuccugca ccaaaagagu aauccuc 27

<210> 178

<211> 27
<212> RNA
<213> Artificial Sequence

<220>
<223> Synthetic oligonucleotide

<400> 178
ucuuccucuu cuuccuccuc cucuccu 27

<210> 179
<211> 27
<212> RNA
<213> Artificial Sequence

<220>
<223> Synthetic oligonucleotide

<400> 179
cagcucuucc ucuucuuccu ccuccuc 27

<210> 180
<211> 27
<212> RNA
<213> Artificial Sequence

<220>
<223> Synthetic oligonucleotide

<400> 180
uugacauccu cugugaggcu caggcuu 27

<210> 181
<211> 27
<212> RNA
<213> Artificial Sequence

<220>
<223> Synthetic oligonucleotide

<400> 181
ggccacuuga cauccucugu gaggcuc 27

<210> 182
<211> 27
<212> RNA
<213> Artificial Sequence

<220>
<223> Synthetic oligonucleotide

<400> 182
ucagagagaa gcucccugaa gccagca 27

<210> 183
<211> 27
<212> RNA
<213> Artificial Sequence

<220>
<223> Synthetic oligonucleotide

<400> 183
agcaggucug gcaggagcug uucgccu 27

<210> 184
<211> 27
<212> RNA
<213> Artificial Sequence

<220>
<223> Synthetic oligonucleotide

<400> 184
aacuugaucu ccaggucggu cagaggc 27

<210> 185
<211> 27
<212> RNA
<213> Artificial Sequence

<220>
<223> Synthetic oligonucleotide

<400> 185
ugaaacuuga ucuccagguc ggucaga 27

<210> 186
<211> 27
<212> RNA
<213> Artificial Sequence

<220>
<223> Synthetic oligonucleotide

<400> 186
guacugaaac uugaucucca ggucggu 27

<210> 187
<211> 27
<212> RNA
<213> Artificial Sequence

<220>
<223> Synthetic oligonucleotide

<400> 187
cgguacugaa acuugaucuc caggucg 27

<210> 188
<211> 27
<212> RNA
<213> Artificial Sequence

<220>
<223> Synthetic oligonucleotide

<400> 188
uggggguugc ugauggugag ggcccgg 27

<210> 189
<211> 27
<212> RNA
<213> Artificial Sequence

<220>
<223> Synthetic oligonucleotide

<400> 189
ccgaagaguu ccaccugcuc cugggug 27

<210> 190
<211> 27
<212> RNA
<213> Artificial Sequence

<220>
<223> Synthetic oligonucleotide

<400> 190
caggcuuaug gggccgaaga guuccac 27

<210> 191
<211> 27
<212> RNA
<213> Artificial Sequence

<220>
<223> Synthetic oligonucleotide

<400> 191
agcugguucg uguagaagcg cugcuug 27

<210> 192
<211> 27
<212> RNA
<213> Artificial Sequence

<220>
<223> Synthetic oligonucleotide

<400> 192
aggacaucca gcagcugguu cguguag 27

<210> 193
<211> 27
<212> RNA
<213> Artificial Sequence

<220>
<223> Synthetic oligonucleotide

<400> 193
uccaggacau ccagcagcug guucgug 27

<210> 194
<211> 27
<212> RNA
<213> Artificial Sequence

<220>
<223> Synthetic oligonucleotide

<400> 194
gcccuguagc uggaggauga gcccgcg 27

<210> 195
<211> 27
<212> RNA
<213> Artificial Sequence

<220>
<223> Synthetic oligonucleotide

<400> 195
uggcccugua gcuggaggau gagcccg 27

<210> 196
<211> 27
<212> RNA
<213> Artificial Sequence

<220>
<223> Synthetic oligonucleotide

<400> 196
uccagaacac cuugcacuga cacaggc 27

<210> 197
<211> 27
<212> RNA
<213> Artificial Sequence

<220>
<223> Synthetic oligonucleotide

<400> 197
uuggucuuga ccucccgcug gaugggg 27

<210> 198
<211> 27
<212> RNA
<213> Artificial Sequence

<220>
<223> Synthetic oligonucleotide

<400> 198
aaaagcuugg ucuugaccuc ccgcugg 27

<210> 199

<211> 27
<212> RNA
<213> Artificial Sequence

<220>
<223> Synthetic oligonucleotide

<400> 199
gcugaaaagc uuggucuuga ccucccg 27

<210> 200
<211> 27
<212> RNA
<213> Artificial Sequence

<220>
<223> Synthetic oligonucleotide

<400> 200
agcucauuga gaaaaugcuc caggcug 27

<210> 201
<211> 27
<212> RNA
<213> Artificial Sequence

<220>
<223> Synthetic oligonucleotide

<400> 201
uuuuggaaca ggaugagcuc auugaga 27

<210> 202
<211> 27
<212> RNA
<213> Artificial Sequence

<220>
<223> Synthetic oligonucleotide

<400> 202
uggcccuuuu ggaacaggau gagcuca 27

<210> 203
<211> 27
<212> RNA
<213> Artificial Sequence

<220>
<223> Synthetic oligonucleotide

<400> 203
aagcagaaga agaucucgaa gggtuggu 27

<210> 204
<211> 27
<212> RNA
<213> Artificial Sequence

<220>
<223> Synthetic oligonucleotide

<400> 204
augagcuucu ucucucgggg uuugcgg 27

<210> 205
<211> 27
<212> RNA
<213> Artificial Sequence

<220>
<223> Synthetic oligonucleotide

<400> 205
guacaguaau gagcuucuuc ucucggg 27

<210> 206
<211> 27
<212> RNA
<213> Artificial Sequence

<220>
<223> Synthetic oligonucleotide

<400> 206
caccuguaca guaaugagcu ucuucuc 27

<210> 207
<211> 27
<212> RNA
<213> Artificial Sequence

<220>
<223> Synthetic oligonucleotide

<400> 207
aacaucucca gcagcagucg agcugcu 27

<210> 208
<211> 27
<212> RNA
<213> Artificial Sequence

<220>
<223> Synthetic oligonucleotide

<400> 208
uacuaucagc ugaccaagau agcuccc 27

<210> 209
<211> 27
<212> RNA
<213> Artificial Sequence

<220>
<223> Synthetic oligonucleotide

<400>  209
ggauacuauc agcugaccaa gauagcu                                         27

<210>  210
<211>  27
<212>  RNA
<213>  Artificial Sequence

<220>
<223>  Synthetic oligonucleotide

<400>  210
gaucuguagc cggauacuau cagcuga                                         27

<210>  211
<211>  27
<212>  RNA
<213>  Artificial Sequence

<220>
<223>  Synthetic oligonucleotide

<400>  211
gagaucugua gccggauacu aucagcu                                         27

<210>  212
<211>  27
<212>  RNA
<213>  Artificial Sequence

<220>
<223>  Synthetic oligonucleotide

<400>  212
uugagaucug uagccggaua cuaucag                                         27

<210>  213
<211>  27
<212>  RNA
<213>  Artificial Sequence

<220>
<223>  Synthetic oligonucleotide

<400>  213
gaggucuggg uuugagaucu guagccg                                         27

<210>  214
<211>  27
<212>  RNA
<213>  Artificial Sequence

<220>
<223>  Synthetic oligonucleotide

<400>  214
gcuccuugaa uugcuccacc augcggu                                         27

<210> 215
<211> 27
<212> RNA
<213> Artificial Sequence

<220>
<223> Synthetic oligonucleotide

<400> 215
agcuccuuga auugcuccac caugcgg 27

<210> 216
<211> 27
<212> RNA
<213> Artificial Sequence

<220>
<223> Synthetic oligonucleotide

<400> 216
cugccagaug ugauggagcu ccuugaa 27

<210> 217
<211> 27
<212> RNA
<213> Artificial Sequence

<220>
<223> Synthetic oligonucleotide

<400> 217
uuguuauugc augccagcug ggugcau 27

<210> 218
<211> 27
<212> RNA
<213> Artificial Sequence

<220>
<223> Synthetic oligonucleotide

<400> 218
gccuuguuau ugcaugccag cugggug 27

<210> 219
<211> 27
<212> RNA
<213> Artificial Sequence

<220>
<223> Synthetic oligonucleotide

<400> 219
cagccuuguu auugcaugcc agcuggg 27

<210> 220

<211> 27
<212> RNA
<213> Artificial Sequence

<220>
<223> Synthetic oligonucleotide

<400> 220
gcagccuugu uauugcaugc cagcugg 27

<210> 221
<211> 27
<212> RNA
<213> Artificial Sequence

<220>
<223> Synthetic oligonucleotide

<400> 221
gggcugucac aucuccacau caguccg 27

<210> 222
<211> 27
<212> RNA
<213> Artificial Sequence

<220>
<223> Synthetic oligonucleotide

<400> 222
caggaaaccc agagguagga cccugca 27

<210> 223
<211> 27
<212> RNA
<213> Artificial Sequence

<220>
<223> Synthetic oligonucleotide

<400> 223
gcccaaaucc acuuccagga aacccag 27

<210> 224
<211> 27
<212> RNA
<213> Artificial Sequence

<220>
<223> Synthetic oligonucleotide

<400> 224
uccuucuugg cccaaaucca cuuccag 27

<210> 225
<211> 27
<212> RNA
<213> Artificial Sequence

<220>
<223> Synthetic oligonucleotide

<400> 225
gggagaguuc uuucccugcu cauggcu 27

<210> 226
<211> 27
<212> RNA
<213> Artificial Sequence

<220>
<223> Synthetic oligonucleotide

<400> 226
uccuccuaua cagcuaggcc ccagggu 27

<210> 227
<211> 27
<212> RNA
<213> Artificial Sequence

<220>
<223> Synthetic oligonucleotide

<400> 227
cuggcuuuug uugccagagg aaauggg 27

<210> 228
<211> 27
<212> RNA
<213> Artificial Sequence

<220>
<223> Synthetic oligonucleotide

<400> 228
gaggacaggg agaugaggaa gugaguc 27

<210> 229
<211> 27
<212> RNA
<213> Artificial Sequence

<220>
<223> Synthetic oligonucleotide

<400> 229
acucauauua ucucagagga cagggag 27

<210> 230
<211> 27
<212> RNA
<213> Artificial Sequence

<220>
<223> Synthetic oligonucleotide

<400> 230
agcaucugau augauaccua agugcuc 27

<210> 231
<211> 27
<212> RNA
<213> Artificial Sequence

<220>
<223> Synthetic oligonucleotide

<400> 231
gcugccagcc uugagcaucu gauauga 27

<210> 232
<211> 27
<212> RNA
<213> Artificial Sequence

<220>
<223> Synthetic oligonucleotide

<400> 232
agguucuugg acucucaaga agggggu 27

<210> 233
<211> 27
<212> RNA
<213> Artificial Sequence

<220>
<223> Synthetic oligonucleotide

<400> 233
ccagguucuu ggacucucaa gaagggg 27

<210> 234
<211> 27
<212> RNA
<213> Artificial Sequence

<220>
<223> Synthetic oligonucleotide

<400> 234
aauuauuucu gcuccagguu cuuggac 27

<210> 235
<211> 27
<212> RNA
<213> Artificial Sequence

<220>
<223> Synthetic oligonucleotide

<400> 235
uaaaaauuau uucugcucca gguucuu 27

<210> 236
<211> 27
<212> RNA
<213> Artificial Sequence

<220>
<223> Synthetic oligonucleotide

<400> 236
aauacauaaa aauuauuucu gcuccag 27

<210> 237
<211> 27
<212> RNA
<213> Artificial Sequence

<220>
<223> Synthetic oligonucleotide

<400> 237
agucuguuuu uaacauucau uaaucca 27

<210> 238
<211> 2771
<212> DNA
<213> Homo sapiens

<400> 238
gtccagctgc gcctggaaag cgagctcgga cccctctgcc atgaaccagt ccatcccagt 60

ggctcccacc ccaccccgcc gcgtgcggct gaagccctgg ctggtggccc aggtgaacag 120

ctgccagtac ccagggcttc aatgggtcaa cggggaaaag aaattattct gcatcccctg 180

gaggcatgcc acaaggcatg gtcccagcca ggacggagat aacaccatct tcaaggcctg 240

ggccaaggag acagggaaat acaccgaagg cgtggatgaa gccgatccgg ccaagtggaa 300

ggccaacctg cgctgtgccc ttaacaagag ccgggacttc cgcctcatct acgacgggcc 360

ccgggacatg ccacctcagc cctacaagat ctacgaggtc tgctccaatg gccctgctcc 420

cacagactcc cagcccccctg aggattactc ttttggtgca ggagaggagg aggaagaaga 480

ggaagagctg cagaggatgt tgccaagcct gagcctcaca gaggatgtca agtggccgcc 540

cactctgcag ccgcccactc tgcggccgcc tactctgcag ccgcccactc tgcagccgcc 600

cgtggtgctg ggtccccctg ctccagaccc cagcccccctg gctcctcccc ctggcaaccc 660

tgctggcttc agggagcttc tctctgaggt cctggagcct gggcccctgc ctgccagcct 720

gccccctgca ggcgaacagc tcctgccaga cctgctgatc agcccccaca tgctgcctct 780

gaccgacctg gagatcaagt ttcagtaccg gggggcggcca ccccgggccc tcaccatcag 840

caacccccat ggctgccggc tcttctacag ccagctggag gccacccagg agcaggtgga 900

actcttcggc cccataagcc tggagcaagt gcgcttcccc agccctgagg acatccccag 960

tgacaagcag cgcttctaca cgaaccagct gctggatgtc ctggaccgcg ggctcatcct 1020

ccagctacag ggccaggacc tttatgccat ccgcctgtgt cagtgcaagg tgttctggag 1080

cgggccttgt gcctcagccc atgactcatg ccccaacccc atccagcggg aggtcaagac 1140

caagcttttc agcctggagc attttctcaa tgagctcatc ctgttccaaa agggccagac 1200

caacacccca ccacccttcg agatcttctt ctgctttggg gaagaatggc ctgaccgcaa 1260

accccgagag aagaagctca ttactgtaca ggtggtgcct gtagcagctc gactgctgct 1320

ggagatgttc tcaggggagc tatcttggtc agctgatagt atccggctac agatctcaaa 1380

cccagacctc aaagaccgca tggtggagca attcaaggag ctccatcaca tctggcagtc 1440

ccagcagcgg ttgcagcctg tggcccaggc ccctcctgga gcaggccttg gtgttggcca 1500

ggggccctgg cctatgcacc cagctggcat gcaataacaa ggctgcagac ggtgactggc 1560

cctggcttcc tgggtggcgg tgcggactga tgtggagatg tgacagcccc gatgagcacc 1620

tggctggctg cagggtccta cctctgggtt tcctggaagt ggatttgggc caagaaggag 1680

agggagaaag gcccgagccc ctgccttccc gggcctttct ctcctgggct gtctctggtc 1740

tggtcagcct ggctctcggg aaattcagcc atgagcaggg aaagaactct cccaaccctg 1800

gggcctagct gtataggagg aattgcctaa gggtggccca ctcttgtgat tgccccattt 1860

cctctggcaa caaaagccag agtgttgtgg gccaagtccc cccacagggc ctctgcaggg 1920

catggccctg atttccctgg tttgagactc acttcctcat ctccctgtcc tctgagataa 1980

tatgagtgag cacttaggta tcatatcaga tgctcaaggc tggcagctac cccttcttg 2040

agagtccaag aacctggagc agaaataatt tttatgtatt tttggattaa tgaatgttaa 2100

aaacagactc agctgtttct ttccttttac tactaccagt tgctcccatg ctgctccacc 2160

aggccctgtt tcggatgcca actggcccac tccccaagca cttgccccca gcttgcgacc 2220

attggcactg ggagggcctg gcttctgggc tgatgggtca gttgggcctt cataaacact 2280

cacctggctg gctttgcctt ccaggaggaa gctggctgaa gcaagggtgt ggaattttaa 2340

atgtgtgcac agtctggaaa actgtcagaa tcagttttcc cataaaaggg tgggctagca 2400

ttgcagctgc atttgggacc attcaaatct gtcactctct tgtgtatatt cctgtgctat 2460

taaatatatc agggcagtgc atgtaaatca tcctgatata tttaatatat ttattatatt 2520

gtcccccgag gtggggacag tgagtgagtt ctcttagtcc ccccagagct ggttgttaaa 2580

gagcctggca cctacccgct ctcacttcat ctgtgtcatc tctgcacact ccagcccact 2640

ttctgccttc agccattgag tggaagctgc cccaggccct taccaggtgc agatgcccaa 2700

tcttgatgcc cagccatcag aactgtgagc caaataaacc tttttctgta taaaaaaaaa 2760

aaaaaaaaaa a 2771

## Description

[Technical Field]

The present invention relates to a nucleic acid for use for suppression of expression of IRF5 or a pharmaceutical composition comprising the nucleic acid.

[Background Art]

Inflammation reaction has an important function in protecting the body when the body is infected with bacterium or virus, or when tissue is injured. On the other hand, excessive inflammation is known to cause autoimmune diseases such as systemic lupus erythematosus, rheumatoid arthritis and the like. Macrophage as one of the immunocytes evokes or suppresses inflammation as its role, and IRF5 (Interferon Regulatory Factor 5) has been identified as a switch that controls inflammatory effects of macrophage. It has been shown that inflammation is promoted by generating more amounts of IRF5 in human macrophage by using viruses, and inflammation is suppressed by suppressing generation of IRF5 by siRNA. It is also known that the amount of transmitters that promote inflammation decreases when a mouse incapable of generating IRF5 due to gene deletion mutation is used (patent document 1, non-patent document 1).

IRF5 is a transcription factor, which is phosphorylated on viral infection and TLR7/8/9 stimulation, localized in the nucleus, and induces many genes such as type I interferon (IFN, IFN$\alpha$, $\beta$ and the like), IL-6, IL-12, TNF$\alpha$, chemokines, apoptosis-related genes and the like, (non-patent document 2). Type I interferon is expressed at high concentration in the serum of systemic lupus erythematosus patients, thereby suggesting the association between IRF5 and systemic lupus erythematosus (non-patent documents 3-7).

As a method of suppressing the expression of IRF5, a method utilizing RNA interference (hereinafter to be also referred to as RNAi) and the like are known. For example, it is known that expression of IRF5 is suppressed by introducing a double stranded RNA having a length of 21 - 23 bases into the cells. For example, a double stranded RNA having a length of 21 - 23 bases that suppress expression of protein and the like by RNA interference has been named as a small interfering RNA (siRNA). While the siRNA sequences targeting human irf5 gene have been partly disclosed (patent document 2, patent document 3, non-patent documents 8-12), their sequences are different from the double stranded RNA of the present invention.

[Document List]

[Patent Documents]

[patent document 1] WO 2012/093258
[patent document 2] WO 2012/005898
[patent document 3] WO 2005/018534


[non-patent documents]

[non-patent document 1] Nat. Immun., 12(3), 231-238 (2011)
[non-patent document 2] Genes. Immun., 8, 445-455 (2007)
[non-patent document 3] Nat. Genet., 38, 550-555 (2006)
[non-patent document 4] Proc. Natl. Acad. Sci. U.S.A., 104, 6758-6763 (2007)
[non-patent document 5] Arthritis. Rheum., 56, 1234-1241 (2007)
[non-patent document 6] Arthritis. Rheum., 58, 826-834 (2008)
[non-patent document 7] Arthritis. Rheum., 60, 1845-1850 (2009)
[non-patent document 8] J. Biol. Chem., 280(17), 17005-17012 (2005)
[non-patent document 9] Cancer Res., 65(16), 7403-7412 (2005)
[non-patent document 10] Blood, 115(22), 4421-4430 (2010)
[non-patent document 11] Nat. Immunol., 12(3), 231-238 (2011)
[non-patent document 12] J. Virol., 79(18), 11671-11676 (2005)

[SUMMARY OF THE INVENTION]

[Problems to be Solved by the Invention]

The present invention aims to provide a nucleic acid capable of suppressing expression of IRF5 that functions as an interferon regulatory factor. The present invention also aims to provide a pharmaceutical composition for the treatment or prophylaxis of autoimmune diseases such as systemic lupus erythematosus, rheumatoid arthritis and the like.

[Means of Solving the Problems]

The present invention relates to the following (1) - (17).

(1) A double-stranded nucleic acid that decreases expression of irf5 gene, which consists of a sense strand and an antisense strand, and comprises a double-stranded region of at least 11 base pairs, wherein an oligonucleotide chain having a chain length of at least 17 nucleotides and 30 nucleotides at most in the aforementioned antisense strand is complementary to a target IRF5 mRNA sequence selected from the group consisting of SEQ ID NOs: 1 - 79.
(2) The double-stranded nucleic acid of (1), wherein the aforementioned double-stranded region is composed of 17 - 27 base pairs, and the 2nd nucleotide from the 5'-terminus of the aforementioned antisense strand complementary to the target IRF5 mRNA sequence selected from the group consisting of SEQ ID NOs: 1 - 79 is complement to the 2nd deoxyribonucleotide from the 3'-terminus of the target IRF5 mRNA sequence.
(3) The double-stranded nucleic acid of (1), wherein the 1st and 2nd nucleotides from the 3'-terminus of an oligonucleotide chain of the aforementioned sense strand are deoxyribonucleotides.
(4) The double-stranded nucleic acid of (1), wherein the 3'-terminus of the aforementioned sense strand and the 5'-terminus of the aforementioned antisense strand form a blunt end.
(5) The double-stranded nucleic acid of (1), wherein the aforementioned sense strand is 25 nucleotides in length and the aforementioned antisense strand is 27 nucleotides in length.
(6) The double-stranded nucleic acid of (1), wherein the aforementioned antisense strand comprises a sequence selected from the group consisting of SEQ ID NOs: 159 - 237.
(7) The double-stranded nucleic acid of (1), wherein the aforementioned sense strand comprises a sequence selected from the group consisting of SEQ ID NOs: 80 - 158.
(8) The double-stranded nucleic acid of (1), comprising one pair of the sense strand/antisense strand sequences, which is selected from the group consisting of SEQ ID NO: 83/SEQ ID NO: 162, SEQ ID NO: 85/SEQ ID NO: 164, SEQ ID NO: 86/SEQ ID NO: 165, SEQ ID NO: 88/SEQ ID NO: 167, SEQ ID NO: 97/SEQ ID NO: 176, SEQ ID NO: 99/SEQ ID NO: 178, SEQ ID NO: 100/SEQ ID NO: 179, SEQ ID NO: 101/SEQ ID NO: 180, SEQ ID NO: 104/SEQ ID NO: 183, SEQ ID NO: 105/SEQ ID NO: 184, SEQ ID NO: 106/SEQ ID NO: 185, SEQ ID NO: 107/SEQ ID NO: 186, SEQ ID NO: 108/SEQ ID NO: 187, SEQ ID NO: 110/SEQ ID NO: 189, SEQ ID NO: 112/SEQ ID NO: 191, SEQ ID NO: 117/SEQ ID NO: 196, SEQ ID NO: 118/SEQ ID NO: 197, SEQ ID NO: 119/SEQ ID NO: 198, SEQ ID NO: 120/SEQ ID NO: 199, SEQ ID NO: 121/SEQ ID NO: 200, SEQ ID NO: 124/SEQ ID NO: 203, SEQ ID NO: 126/SEQ ID NO: 205, SEQ ID NO: 127/SEQ ID NO: 206, SEQ ID NO: 128/SEQ ID NO: 207, SEQ ID NO: 129/SEQ ID NO: 208, SEQ ID NO: 130/SEQ ID NO: 209, SEQ ID NO: 131/SEQ ID NO: 210, SEQ ID NO: 132/SEQ ID NO: 211, SEQ ID NO: 133/SEQ ID NO: 212, SEQ ID NO: 134/SEQ ID NO: 213, SEQ ID NO: 138/SEQ ID NO: 217, SEQ ID NO: 147/SEQ ID NO: 226, SEQ ID NO: 149/SEQ ID NO: 228, SEQ ID NO: 150/SEQ ID NO: 229, SEQ ID NO: 151/SEQ ID NO: 230, SEQ ID NO: 155/SEQ ID NO: 234, SEQ ID NO: 156/SEQ ID NO: 235, and SEQ ID NO: 157/SEQ ID NO: 236.
(9) A pharmaceutical composition comprising the double stranded nucleic acid of any one of (1) - (8).
(10) A method of treating an autoimmune disease, comprising a step of administering a therapeutically effective amount of the double stranded nucleic acid of any one of (1) - (8) or the pharmaceutical composition of (9) to a human in need of the treatment.
(11) The method of (10), wherein the autoimmune disease is systemic lupus erythematosus and/or rheumatoid arthritis.
(12) The pharmaceutical composition of (9), which is for the treatment of an autoimmune disease.
(13) The pharmaceutical composition of (12), wherein the autoimmune disease is systemic lupus erythematosus and/or rheumatoid arthritis.
(14) The double stranded nucleic acid of any of (1) - (8), which is for use for the treatment of an autoimmune disease.
(15) The double stranded nucleic acid of (14), wherein the autoimmune disease is systemic lupus erythematosus and/or rheumatoid arthritis.
(16) Use of the double stranded nucleic acid of any of (1) - (8) in the production of a therapeutic agent for an autoimmune disease.

(17) The use of (16), wherein the autoimmune disease is systemic lupus erythematosus and/or rheumatoid arthritis.

[Effect of the Invention]

Expression of an interferon regulatory factor can be suppressed by providing the nucleic acid of the present invention having an IRF5 expression suppressing activity, a vector encoding the nucleic acid, or a pharmaceutical composition comprising the nucleic acid or the vector. The present invention is particularly useful for the treatment and/or prophylaxis of autoimmune diseases such as systemic lupus erythematosus, rheumatoid arthritis and the like.

[Description of Embodiments]

As an irf5 gene (gene encoding IRF5) targeted by the nucleic acid of the present invention, for example, a gene producing a full-length mRNA of irf5 corresponding to IRF5 cDNA (SEQ ID NO: 238) registered as Genbank Accession No.NM_032643 can be mentioned.

1. nucleic acid of the present invention

In the present invention, a nucleic acid comprising a nucleotide sequence complementary to IRF5 mRNA is referred to as an antisense strand nucleic acid, and a nucleic acid comprising a nucleotide sequence complementary to a nucleotide sequence of an antisense strand nucleic acid is also referred to as a sense strand nucleic acid. In the present specification, unless otherwise specified, "the nucleic acid of the present invention" is used to encompass antisense strand nucleic acid, sense strand nucleic acid, and double-stranded nucleic acid pairing a sense strand and an antisense strand nucleic acid.

The nucleic acid of the present invention may be any molecule as long as it is a molecule wherein nucleotide or molecule having equivalent function as that of the nucleotide are polymerized. Examples of thereof include RNA which is a polymer of ribonucleotide, DNA which is a polymer of deoxyribonucleotide, chimeric nucleic acid composed of RNA and DNA, and nucleotide polymer wherein at least one nucleotide of these nucleic acids is substituted by a molecule having equivalent function as that of nucleotide. In addition, a derivative containing at least one molecule having equivalent function as that of the nucleotide in these nucleic acids is also encompassed in the nucleic acid of the present invention. Uracil (U) can be unambiguously read as thymine (T).

Examples of the molecule having equivalent function as that of the nucleotide include nucleotide derivatives and the like. The nucleotide derivative may be any molecule as long as it is a molecule obtained by modifying nucleotide. For example, a molecule obtained by modifying ribonucleotide or deoxyribonucleotide and the like to improve or stabilize nuclease resistance, enhance affinity for complementary chain nucleic acid, enhance cell permeability or visualize same, as compared to RNA or DNA, are preferably used.

Examples of the molecule obtained by modifying a nucleotide include sugar moiety-modified nucleotide, phosphodiester bond-modified nucleotide, base-modified nucleotide, a nucleotide wherein at least one of a sugar moiety, a phosphodiester bond and a base is modified and the like.

While the sugar moiety-modified nucleotide may be any as long as the chemical structure of sugar of nucleotide is partly or entirely modified or substituted by any substituent, or substituted by any atom, 2'-modified nucleotide is preferably used.

Examples of the 2'-modified nucleotide include a nucleotide wherein 2'-OH group of ribose is substituted by a substituent selected from H, OR, R, R'OR, SH, SR, $NH_2$, NHR, $NR_2$, $N_3$, CN, F, Cl, Br and I (R is alkyl or aryl, preferably alkyl having 1 - 6 carbon atoms, R' is alkylene, preferably alkylene having 1 - 6 carbon atoms), preferably a nucleotide wherein 2'-OH group is substituted by H, F or methoxy group, more preferably a nucleotide wherein 2'-OH group is substituted by F or methoxy group. In addition, a nucleotide wherein 2'-OH group is substituted by a substituent selected from the group consisting of 2-(methoxy)ethoxy group, 3-aminopropoxy group, 2-[(N,N-dimethylamino)oxy]ethoxy group, 3-(N,N-dimethylamino)propoxy group, 2-[2-(N,N-dimethylamino)ethoxy]ethoxy group, 2-(methylamino)-2-oxoethoxy group, 2-(N-methylcarbamoyl)ethoxy group and 2-cyanoethoxy group, and the like can also be mentioned.

As the sugar moiety modified nucleotide, a crosslinking structure type artificial nucleic acid having two cyclic structures by introducing a crosslinking structure into the sugar moiety (Bridged Nucleic Acid) (BNA) can be mentioned. Specific examples thereof include locked artificial nucleic acid wherein the 2'-position oxygen atom and the 4'-position carbon atom are crosslinked via methylene (Locked Nucleic Acid) (LNA), ethylene crosslinking structure type artificial nucleic acid (Ethylene bridged nucleic acid) (ENA) [Nucleic Acid Research, 32, e175(2004)] and the like, and further, peptide nucleic acid (PNA) [Acc. Chem. Res., 32, 624 (1999)], oxy-peptide nucleic acid (OPNA) [J. Am. Chem. Soc., 123, 4653 (2001)], peptide ribonucleic acid (PRNA) [J. Am. Chem. Soc., 122, 6900 (2000)] and the like.

The phosphodiester bond-modified nucleotide may be any as long as the chemical structure of the phosphodiester bond is partly or entirely modified or substituted by any substituent, or substituted by any atom. Examples thereof include a nucleotide wherein phosphodiester bond is substituted by phosphorothioate bond, a nucleotide wherein phosphodiester bond is substituted by phosphorodithioate bond, a nucleotide wherein phosphodiester bond is substituted by alkylphosphonate bond, a nucleotide wherein phosphodiester bond is substituted by phosphoramidate bond and the like.

The base-modified nucleotide may be any as long as the chemical structure of the base of the nucleotide is partly or entirely modified or substituted by any substituent, or substituted by any atom. Examples thereof include one wherein oxygen atom in the base is substituted by sulfur atom, one wherein hydrogen atom is substituted by alkyl group having 1-6 carbon atoms, halogen and the like, one wherein methyl group is substituted by hydrogen, hydroxymethyl, alkyl group having 2 - 6 carbon atoms and the like, and one wherein amino group is substituted by alkyl group having 1 - 6 carbon atoms, alkanoyl group having 1 - 6 carbon atoms, oxo group, hydroxy group, and the like.

As the nucleotide derivative, one obtained by adding other chemical substance such as peptide, protein, sugar, lipid, phospholipid, phenazine, folate, phenanthridine, anthraquinone, acridine, fluorescein, rhodamine, coumarin, dye and the like, directly or via a linker, to a nucleotide or a nucleotide derivative wherein at least one of sugar moiety, phosphodiester bond and base is modified can also be mentioned. Specific examples thereof include 5'-polyamine-added nucleotide derivative, cholesterol-added nucleotide derivative, steroid-added nucleotide derivative, bile acid-added nucleotide derivative, vitamin-added nucleotide derivative, Cy5-added nucleotide derivative, Cy3-added nucleotide derivative, 6-FAM-added nucleotide derivative, and biotin-added nucleotide derivative and the like.

The nucleotide derivative may form a crosslinking structure, such as alkylene structure, peptide structure, nucleotide structure, ether structure, ester structure, a structure of a combination of at least one of these and the like, with other nucleotide or nucleotide derivative in the nucleic acid.

The nucleic acid of the present invention also encompasses a nucleic acid wherein the atoms in a molecule are partly or entirely substituted by an atom (isotope) having a different mass number.

In the present specification, "complement" means a relationship forming a base pairing between two bases, and refers to a double helix structure as a whole double-stranded region via a loose hydrogen bond, for example, the relationship between adenine and thymine or uracil, and the relationship between guanine and cytosine.

In the present specification, "complementary" means not only complete complementarity between two nucleotide sequences, but also includes 0 - 30%, 0 - 20% or 0 - 10% of mismatch bases between the nucleotide sequences. For example, an antisense strand complementary to IRF5 mRNA may contain substitution of one or more bases in a nucleotide sequence completely complementary to a partial nucleotide sequence of the mRNA. To be specific, an antisense strand may contain 1 - 8, preferably 1 - 6, 1 - 4, 1 - 3, particularly 2 or one mismatch base in a target sequence of the target gene. For example, when the antisense strand has 27 bases in length, it may contain 8, 7, 6, 5, 4, 3, 2 or one mismatch base in a target sequence of the target gene, and the position of the mismatch may be the 5'-terminus or 3'-terminus of the sequence.

In addition, "complementary" encompasses a nucleotide sequence wherein one of the sequences may be completely complementary to the other nucleotide sequence, and one or more bases are added and/or deleted. To be specific, due to the addition and/or deletion of the bases of the antisense strand, the target IRF5 mRNA sequence may contain 1 or 2 bulge bases.

The nucleic acid of the present invention may be constituted of any nucleotide or a derivative thereof as long as it is a nucleic acid containing a nucleotide sequence complementary to a part of the nucleotide sequence of IRF5 mRNA and/or a nucleic acid containing a nucleotide sequence complementary to the nucleotide sequence of the nucleic acid. The double-stranded nucleic acid of the present invention may have any length as long as a nucleic acid containing a nucleotide sequence complementary to the target IRF5 mRNA sequence and a nucleic acid containing a nucleotide sequence complementary to the nucleotide sequence of the nucleic acid can form a double strand. The length of the sequence forming a double strand is generally 11 - 35 bases, preferably 15 - 30 bases, more preferably 17 - 25 bases, further preferably 17 - 23 bases, particularly preferably 19 - 23 bases.

In one embodiment, the nucleic acid of the present invention may be a Dicer-Substrate siRNA (DsiRNA). Dicer is one of the major factors that function in RNA interference, and processes a double stranded RNA molecule to produce siRNA of 21 bases. The produced siRNA is uptaken into an RISC complex, and the target mRNA molecule is degraded in the complex. DsiRNA is a double stranded RNA optimized for processing by Dicer and uptake by the RISC complex, and has a structure wherein an antisense strand consisting of a ribonucleotide of 27 bases and a sense strand consisting of ribonucleotide and deoxyribonucleotide of 25 bases form a double strand. The deoxyribonucleotide is located in the sense strand at the first and the second nucleotides from the 3'-terminus. DsiRNA produces siRNA (19 base pairs) of 21 bases when processed by Dicer. Since DsiRNA is known to afford a higher effect of RNA interference than siRNA, it can be preferably used as the nucleic acid of the present invention.

As the antisense strand nucleic acid of the present invention, a nucleic acid containing a nucleotide sequence complementary to the target IRF5 mRNA sequence is used, wherein 1 - 3 bases, preferably 1 - 2 bases, more preferably 1 base, in the nucleic acid may be deleted, substituted or added.

As a nucleic acid that suppresses expression of IRF5, a single strand nucleic acid containing a nucleotide sequence complementary to the target IRF5 mRNA sequence and capable of suppressing the expression of IRF5, or a double-stranded nucleic acid consisting of a nucleic acid containing a nucleotide sequence complementary to the target IRF5 mRNA sequence and a nucleic acid containing a nucleotide sequence complementary to the nucleotide sequence of the nucleic acid, and capable of suppressing the expression of IRF5 is preferably used.

In the present invention, a double-stranded nucleic acid refers to a nucleic acid wherein two nucleotide chains are paired to form a double-stranded region. The double-stranded region refers to a portion in which a nucleotide or a derivative thereof constituting a double-stranded nucleic acid constitutes a base pair to form a double strand. The double-stranded region generally contains 11 - 35 base pairs, preferably 15 - 30 base pairs, more preferably 17 - 25 base pairs, further preferably 17 - 23 base pairs, particularly preferably 19 - 23 base pairs.

A single strand nucleic acid constituting a double-stranded nucleic acid generally consists of 11 - 30 bases, preferably 15 - 29 bases, more preferably 15 - 27 bases, further preferably 15 - 25 bases, particularly preferably 17 - 23 bases, most preferably 19 - 21 bases.

When the double-stranded nucleic acid of the present invention has an additional nucleotide or nucleotide derivative that does not form a double strand on the 3'-side or 5'-side following a double-stranded region, it is called a protruding part (overhang). When a protruding part is present, a nucleotide constituting the protruding part may be ribonucleotide, deoxyribonucleotide or a derivative thereof.

As a double-stranded nucleic acid having a protruding part, one having a protruding part of 1 - 6 bases, generally 1 - 3 bases, preferably one having a protruding part of 2 bases, for example, protruding part composed of dTdT or UU, on the 3'-terminus or 5'-terminus of at least one of the chains is used. The protruding part may be present in an antisense strand alone, a sense strand alone, or both an antisense strand and a sense strand. In the present invention, a double-stranded nucleic acid having protruding part in both an antisense strand and a sense strand is preferably used. In the antisense strand, an oligonucleotide chain consisting of at least 17 nucleotides and at most 30 nucleotides and comprising a double-stranded region and a subsequent protruding part is complementary to a target IRF5 mRNA sequence selected from the group described in Table 1. As the double-stranded nucleic acid of the present invention, for example, a nucleic acid molecule generating the above-mentioned double-stranded nucleic acid by the action of a ribonuclease such as Dicer and the like (WO2005/089287), a double-stranded nucleic acid forming a blunt end without having a protruding part on the 3'-terminus or 5'-terminus, a double-stranded nucleic acid with protrusion of a sense strand alone (US2012/0040459) and the like can also be used.

As the double-stranded nucleic acid of the present invention, a nucleic acid consisting of the same sequence as a nucleotide sequence of the target gene or a nucleotide sequence of a complementary chain thereof may be used, or a double-stranded nucleic acid consisting of a nucleic acid wherein 1 - 4 bases on the 5'-terminus or 3'-terminus of at least one of the chains of the nucleic acid is deleted, and a nucleic acid containing a nucleotide sequence complementary to a nucleotide sequence of the nucleic acid may be used.

The double-stranded nucleic acid of the present invention may be a double-stranded RNA (dsRNA) wherein RNAs form a double strand, a double-stranded DNA (dsDNA) wherein DNAs form a double strand, or a hybrid nucleic acid wherein RNA and DNA form a double strand. Alternatively, one or both of the chains of the double strand may be a chimeric nucleic acid of DNA and RNA. Preferred is a double-stranded RNA (dsRNA).

The 2nd nucleotide from the 5'-terminus of the antisense strand of the present invention is preferably complement to the 2nd deoxyribonucleotide from the 3'-terminus of the target IRF5 mRNA sequence, the 2 - 7th from the 5'-terminus of the antisense strand is more preferably completely complement to the 2 - 7th deoxyribonucleotides from the 3'-terminus of the target IRF5 mRNA sequence, and the 2 - 11th from the 5'-terminus of the antisense strand is further preferably completely complement to the 2 - 11th deoxyribonucleotides from the 3'-terminus of the target IRF5 mRNA sequence. In addition, the 11th nucleotide from the 5'-terminus of the antisense strand of the nucleic acid of the present invention is preferably complement to the 11th deoxyribonucleotide from the 3'-terminus of the target IRF5 mRNA sequence, the 9 - 13th nucleotides from the 5'-terminus of the antisense strand is more preferably completely complement to the 9 - 13th from the 3'-terminus of the target IRF5 mRNA sequence, and the 7 - 15th from the 5'-terminus of the antisense strand is further preferably completely complement to the 7 - 15th deoxyribonucleotides from the 3'-terminus of the target IRF5 mRNA sequence.

A method of producing the nucleic acid of the present invention is not particularly limited, and a method using a known chemical synthesis, or an enzymatic transcription method and the like can be mentioned. As a method using a known chemical synthesis, a phosphoramidite method, a phosphorothioate method, a phosphotriester method, a CEM method [Nucleic Acid Research, 35, 3287 (2007)] and the like can be mentioned and, for example, it can be synthesized by ABI3900 High Throughput nucleic acid synthesizer (manufactured by Applied Biosystems). After completion of the synthesis, desorption from a solid phase, removal of a protecting group, purification of the object product and the like are performed. It is desirable to obtain a nucleic acid having purity of not less than 90%, preferably not less than 95%, by purification. In the case of a double-stranded nucleic acid, synthesized and purified sense strand and antisense strand are mixed at a suitable ratio, for example, 0.1 - 10 equivalents, preferably 0.5 - 2 equivalents, more preferably 0.9 - 1.1 equivalents, further preferably an equivalent molar quantity, of sense strand per 1 equivalent of antisense strand, and may be used after annealing, or directly used without a step of annealing the mixture. Annealing may be performed under any conditions as long as a double-stranded nucleic acid can be formed. It is generally performed by mixing almost equivalent molar quantities of sense strand and antisense strand, heating same at about 94°C for about 5 min and slowly cooling to room temperature. As an enzymatic transcription method for producing the nucleic acid of the present invention, a method using a plasmid or DNA having the object nucleotide sequence as a template, and including transcription using phage RNA polymerase, for example, T7, T3, or SP6RNA polymerase, can be mentioned.

The nucleic acid of the present invention can be introduced into a cell by using a carrier for transfection, preferably a cationic carrier such as cationic liposome and the like. Also, it can be directly introduced into a cell by a calcium phosphate method, an electroporation method, a microinjection method and the like.

In the nucleic acid of the present invention, the 5'-terminus, the 3'-terminus and/or an inner portion of sequence may be modified by one or more ligands and fluorophores, and a nucleic acid modified by a ligand or fluorophore is also called a conjugate nucleic acid. It is possible to provide a modification on the 5'-terminus, the 3'-terminus and/or an inner portion of sequence by reacting, during elongation reaction on a solid phase, a modifier capable of reaction on the solid phase. A conjugate nucleic acid can also be obtained by synthesizing and purifying, in advance, a nucleic acid introduced with a functional group such as amino group, mercapto group, azido group, triple bond and the like, and reacting same with a modifier. While the ligand may be a molecule having affinity for a biological molecule, for example, lipids such as cholesterol, fatty acid, tocopherol, retinoid and the like, saccharides such as N-acetylgalactosamine (GalNAc), galactose (Gal), mannose (Man) and the like, antibodies such as full antibody, Fab, VHH and the like, proteins such as low-density lipoprotein (LDL), human serum albumin and the like, peptides such as RGD, NGR, R9, CPP and the like, small molecules such as folic acid and the like, synthesis polymers such as synthetic polyamino acid and the like, nucleic acid aptamers and the like can be mentioned, and these can also be used in combination. Examples of the fluorophore include Cy3 series, Alexa series, black hole quencher and the like.

A vector capable of expressing the nucleic acid of the present invention after introduction into a cell may be used instead of the nucleic acid of the present invention. To be specific, an expression vector is constructed by inserting a sequence encoding the nucleic acid of the present invention into the downstream of a promoter in the expression vector, and introduced into a cell, whereby the nucleic acid and the like can be expressed. Examples of the expression vector include pCDNA6.2-GW/miR (manufactured by Invitrogen), pSilencer 4.1-CMV (manufactured by Ambion), pSINsi-hH1 DNA (manufactured by Takara Bio Inc.), pSINsi-hU6 DNA (manufactured by Takara Bio Inc.), pENTR/U6 (manufactured by Invitrogen) and the like.

It is also possible to use a recombinant viral vector produced by inserting a sequence encoding the nucleic acid of the present invention into the downstream of a promoter in the expression vector and introducing the vector into a packaging cell. Examples of the viral vector include retroviral vector, lentiviral vector, adenoviral vector, adeno-associated viral vector and the like.

2. nucleic acid having activity to suppress expression of IRF5

The antisense strand and sense strand of the present invention can be designed based on, for example, a nucleotide sequence (SEQ ID NO: 238) of cDNA (sense strand) of the full length mRNA of human irf5 registered as Genbank Accession No.NM_032643.

As a nucleic acid having an activity to suppress expression of IRF5, a double-stranded nucleic acid having an activity to suppress expression of IRF5, which consists of the antisense strand nucleic acid of the present invention containing a nucleotide sequence complementary to IRF5 mRNA, and the sense strand nucleic acid of the present invention containing a nucleotide sequence complementary to the nucleotide sequence of the nucleic acid, can be mentioned. A single strand nucleic acid constituting the double-stranded nucleic acid generally consists of 11 - 30 bases, preferably 15 - 29 bases, more preferably 15 - 27 bases, further preferably 15 - 25 bases, particularly preferably 17 - 23 bases, most preferably 19 - 21 bases. The double-stranded nucleic acid has a double-stranded region generally consisting of 11 - 35 base pairs, preferably 15 - 30 base pairs, more preferably 17 - 25 base pairs, further preferably 17 - 23 base pairs, particularly preferably 19 - 23 base pairs.

The expression of IRF5 can be suppressed by introducing these double-stranded nucleic acids into a cell. For example, the double-stranded nucleic acid of the present invention introduced into a cell at a concentration of several hundred pM - several nM can suppress expression of IRF5 mRNA when cultured for not less than 24 hrs, for example, for 48 hrs.

The expression suppressive activity on IRF5 mRNA by the double-stranded nucleic acid of the present invention can be evaluated by transfecting the nucleic acid and the like to a human cell line and the like by using a cationic liposome and the like, culturing same for a given period, and quantifying the expression level of IRF5 mRNA in the human cell line.

3. pharmaceutical composition of the present invention

The present invention also relates to a pharmaceutical composition comprising a nucleic acid such as the above-mentioned double-stranded nucleic acid as an active ingredient. The pharmaceutical composition of the present invention can be used as a therapeutic or prophylactic agent for autoimmune diseases such as systemic lupus erythematosus, rheumatoid arthritis and the like.

The pharmaceutical composition can further comprise a carrier effective for intracellular transfer of the nucleic acid. Examples of the carrier effective for intracellular transfer of the nucleic acid include cationic carriers. Examples of the cationic carrier include a cationic liposome, a cationic polymer and the like. As a carrier effective for intracellular transfer of the nucleic acid, a carrier utilizing a virus envelope may also be used. As a cationic polymer, JetSI (Qbiogene Inc.), Jet-PEI (polyethyleneimine; Qbiogene Inc.) and the like are preferably used. As a carrier utilizing a virus envelope, GenomeOne (HVJ-E liposome; ISHIHARA SANGYO KAISHA, LTD.) and the like are preferably used.

A composition comprising the nucleic acid of the present invention and the above-mentioned carrier can be prepared by a method known to those of ordinary skill in the art. For example, it can be prepared by mixing a carrier dispersion liquid and a nucleic acid solution at suitable concentrations. When a cationic carrier is used, generally, it can be prepared easily by mixing in an aqueous solution by a conventional method, since a nucleic acid has a negative electric charge in aqueous solutions. Examples of the aqueous solvent used for the preparation of the composition include electrolytic solutions such as water for injection, distilled water for injection, saline and the like, sugar solutions such as glucose solution, maltose solution and the like, and the like. The conditions such as pH and temperature and the like for preparation of the composition can be appropriately selected by those of ordinary skill in the art.

Where necessary, the composition can also be formed as a uniform composition by a dispersion treatment using an ultrasonic dispersion apparatus, a high-pressure emulsion apparatus and the like. Since the method and conditions optimal for the preparation of a composition comprising a carrier and a nucleic acid depend on the carrier to be used, those of ordinary skill in the art can select an optimal method for the carrier to be used irrespective of the above-mentioned methods.

As the pharmaceutical composition of the present invention, a composition constituted of a composite particle comprising a nucleic acid and a lead particle as constituent components and a lipid membrane covering the composite particle can also be used preferably. Examples of the lead particle include a lipid assembly, a liposome, an emulsion particle, a polymer, a metal colloid, a fine particle preparation and the like, and a liposome is preferably used. The lead particle in the present invention may contain a complex of a combination of not less than two from a lipid assembly, a liposome, an emulsion particle, a polymer, a metal colloid, a fine particle preparation and the like as a constituent component, or a complex of a combination of a lipid assembly, a liposome, an emulsion particle, a polymer, a metal colloid, a fine particle preparation and the like and other compound (e.g., sugar, lipid, inorganic compound etc.) as a constituent component.

Examples of the lipid membrane covering the composite particle include those comprising non-cationic lipid, lipid suppressing aggregation of particles and cationic lipid and the like as a constituent component.

The composition can be prepared according to, for example, the method described in WO 2006/080118 and the like.

A suitable mixing ratio of the nucleic acid and the carrier comprised in the pharmaceutical composition of the present invention is 1 - 200 parts by weight of a carrier per 1 part by weight of nucleic acid. It is preferably 2.5 - 100 parts by weight, further preferably 7 - 25 parts by weight, of a carrier per 1 part by weight of a nucleic acid.

An average particle size of the pharmaceutical composition of the present invention is preferably about 10 nm - 300 nm, more preferably about 30 nm - 200 nm, further preferably about 50 nm - 150 nm.

The pharmaceutical composition of the present invention may also comprise a pharmaceutically acceptable carrier, a diluent and the like besides the above-mentioned carrier. A pharmaceutically acceptable carrier, a diluent and the like are essentially chemically-inactive and harmless compositions, and do not at all influence the biological activity of the pharmaceutical composition of the present invention. Examples of the carrier and diluent include, but are not limited to, a salt solution, a sugar solution, a glycerol solution, ethanol and the like.

The pharmaceutical composition of the present invention comprises the complex in an amount effective for the treatment or prevention of diseases and is provided in a form permitting appropriate administration to patients. The formulation of the pharmaceutical composition of the present invention may be, for example, a liquid such as injection, eye drop, inhalation and the like, for example, an external preparation such as ointment, lotion and the like.

In the case of a liquid, the concentration range of the active ingredient in the pharmaceutical composition of the present invention is generally 0.001 - 25%(w/v), preferably 0.1 - 10%(w/v), more preferably 0.5 - 5%(w/v). The pharmaceutical composition of the present invention may comprise an adequate amount of any pharmaceutically acceptable additive, for example, an emulsion adjuvant, a stabilizer, an isotonicifier, a pH adjuster and the like. Any pharmaceutically acceptable additive can be added in a suitable step before or after dispersion of the complex.

The pH of the solution is generally adjusted to about 5.0 - about 8.5, preferably about 6.0 - about 8.0, and preferably subjected to a sterilization treatment such as sterilization by filtration and the like, by using a membrane filter and the like.

The pharmaceutical composition of the present invention can also be prepared as a freeze-dried preparation.

A freeze-dried preparation can be prepared by a dispersion treatment of a nucleic acid and a carrier, followed by a freeze-drying treatment. A freeze-drying treatment can be performed by a conventional method. For example, a given amount of a complex solution after the above-mentioned dispersion treatment is dispensed in a vial container under sterile conditions, predried for about 2 hrs under the condition of about -40°C to -20°C, primarily predried at about 0 - 10°C under reduced pressure, then secondarily dried at about 15 - 25°C under reduced pressure to perform freeze-drying. Then, for example, the inside of the vial is substituted with a nitrogen gas and a cap is provided, whereby a freeze-dried preparation of the pharmaceutical composition of the present invention can be obtained.

The freeze-dried preparation can be used by redissolving by the addition of any suitable solution. Examples of the solution include electrolytic solutions such as water for injection, saline and the like, glucose solution, other general infusions and the like. While the liquid volume of this solution varies depending on the use and the like and is not particularly limited, it is preferably a 0.5- to 2-fold amount of the liquid volume before freeze-drying, or not more than 500 ml.

The pharmaceutical composition of the present invention can be administered to animals including human by, for example, intravenous administration, intraarterial administration, oral administration, tissue administration, transdermal administration, transmucosal administration or rectal administration, and is preferably administered by an appropriate method according to the symptom of the patient. Particularly, intravenous administration, transdermal administration, and transmucosal administration are preferably used. In addition, topical administration such as topical administration to a cancer site and the like can also be employed. Examples of the dosage form suitable for these administration methods include various injections, oral preparations, drip infusions, absorbents, eye drops, ointments, lotions, suppositories and the like.

While the dose of the pharmaceutical composition of the present invention is desirably determined in consideration of drug, dosage form, condition of patient such as age, body weight and the like, administration route, nature and severity of the disease and the like, it is generally 0.1 mg - 10 g/day, preferably 1 mg - 500 mg/day, for an adult in the mass of the nucleic acid. In some cases, a dose below these levels may be sufficient, or a dose above these levels may be conversely required. The pharmaceutical composition can be administered one to several times per day, or can be administered at one to several day intervals.

4. treatment method

The treatment method of the disease of the present invention is a method of treating an autoimmune disease, comprising administering a therapeutically effective amount of the nucleic acid of the present invention or the pharmaceutical composition of the present invention to a human in need of the treatment. Other steps and conditions are not limited in any manner.

The treatment method of the present invention can quote, for example, the aforementioned administration method, dose, preparation method and the like of the pharmaceutical composition of the present invention.

In the present specification, the autoimmune disease includes systemic autoimmune diseases and organ-specific autoimmune diseases. Examples of the autoimmune disease include systemic autoimmune diseases such as rheumatoid arthritis, systemic lupus erythematosus, antiphospholipid antibody syndrome, IgG4-related disease, polymyositis, dermatomyositis, scleroderma, Sjogren's syndrome, vasculitis syndrome and mixed connective tissue disease and the like, and organ-specific autoimmune diseases such as Guillain-Barre syndrome, myasthenia gravis, chronic gastritis, chronic atrophic gastritis, autoimmune hepatitis, primary biliary cirrhosis, ulcerative colitis, Crohn's disease, primary sclerosing cholangitis, autoimmune pancreatitis, aortitis syndrome, Goodpasture's syndrome, rapidly progressive glomerulonephritis, megaloblastic anemia, autoimmune hemolytic anemia, autoimmune neutropenia, idiopathic thrombocytopenic purpura, Basedow disease, Hashimoto's disease, primary hypothyroidism, idiopathic Addison's disease, type 1 diabetes, chronic discoid lupus erythematosus, circumscribed scleroderma, pemphigus, pemphigoid, herpes gestationis, linear IgA bullous dermatosis, acquired epidermolysis bullosa, circular shape alopecia, vitiligo vulgaris, Sutton's Leukoderma acquisitum centrifugum.Sutton's nevus, Harada disease, Autoimmune optic neuropathy, autoimmune inner ear disease, idiopathic azoospermia and habitual abortion and the like. Preferably, the autoimmune disease includes rheumatoid arthritis and systemic lupus erythematosus.

The present invention is explained in the following by referring to Examples, which are not to be construed as limitative.

[Examples]

Example 1: Preparation of double stranded nucleic acid

Sense strands (SEQ ID NOs: 80 - 158), antisense strands (SEQ ID NOs: 159 - 237) and double stranded nucleic acid DsiKKC-01 - 79 obtained by annealing them, which are shown in Table 1, were synthesized by IDT (Integrated DNA Technologies, Inc.) under commitment. In Table 1, uppercase letters show ribonucleotides, and lower case letters show deoxyribonucleotides.

Example 2: Measurement of knockdown activity of IRF5 mRNA

The double stranded nucleic acids described in Table 1 and Dharmafect 1 siRNA transfection reagent (manufactured by Thermo Fisher Scientific, catalog No.: T-2001) were diluted with Opti-MEM medium (manufactured by Life Technologies, catalog No. 11058-021) to prepare siRNA/Dharmafect 1 mixture of double stranded nucleic acid at a final concentration of 10 nM and a 0.5% Dharmafect 1 siRNA transfection reagent. 50 μL of each siRNA/Dharmafect 1 mixture was dispensed to a 96-well culture plate, THP-1 cells (ATCC catalog No. TIB-202), which are human immunocytes derived from leukemia, were seeded in each well at 50,000 cell number/50 μL/well, and cultured under the conditions of 37°C, 5% $CO_2$ for 2 hr. Thereafter, the culture supernatant was removed, and the cells were resuspended in a fresh complete medium [RPMI medium (manufactured by Life Technologies, catalog No. 11875-093) containing 10% fetal bovine serum (FBS)], and further incubated under the conditions of 37°C, 5% $CO_2$ for 2 days. The amount of IRF5 mRNA in the THP-1 cells was quantified using Affymetrix Quantigene 2.0 (manufactured by Affymetrix, catalog No. #12773) and according to the method described in the manual attached to the product. For quantification, a probe for hybridizing to IRF5 mRNA (manufactured by Affymetrix, catalog No. #SA-50356) and a probe for hybridizing to PPIB mRNA (manufactured by Affymetrix, catalog No. #SA-50205) were used.

Table 2 shows a IRF5 mRNA knockdown rate by each double stranded nucleic acid. Mock shows an mRNA knockdown rate when THP-1 cells were treated with a transfection reagent alone without addition of siRNA. mRNA knockdown rate was calculated by multiplying the value calculated according to following formula by 100. An IRF5 mRNA knockdown activity by the double stranded nucleic acid described in Table 1 was observed. Particularly, DsiKKC-28, DsiKKC-29, DsiKKC-38 and DsiKKC-42 showed a high knockdown activity.

$$\text{mRNA knockdown rate}=1-\{[(IRF5_{testsiRNA}-IRF5_{background})/(PPIB_{testsiRNA}-PPIB_{background})]/[(IRF5_{siKKC3}-IRF5_{background})/(PPIB_{siKKC3}-PPIB_{background})]\}$$

As an internal control, a constitutively expressed gene PPIB (peptidylprolyl isomerase B) was used. As a negative control, siKKC3 (manufactured by Qiagen, catalog No. #1027280) that does not intersect with any of the human genes was used. The quantified value in each reaction system after a similar operation except that THP-1 cells were not used was subtracted from the quantified value in each reaction system for background normalization.

[Table 1-1]

| double stranded nucleic acid | target IRF5 cDNA. sequence | SEQ ID NO: | sense strand (5' - 3') | SEQ ID NO: | antisense strand (5' - 3') | SEQ ID NO: |
|---|---|---|---|---|---|---|
| DsiKKC-01 | CCAGTCCATCCCAGTGGCTCCCACC | 1 | CCAGUCCAUCCCAGUGGCUCCCAcc | 80 | GGUGGGAGCCACUGGGAUGGACUGGUU | 159 |
| DsiKKC-02 | GGCTGGTGGCCCAGGTGAACAGCTG | 2 | GGCUGGUGGCCCAGGUGAACAGCtg | 81 | CAGCUGUUCACCUGGGCCACCAGCCAG | 160 |
| DsiKKC-03 | CAGGTGAACAGCTGCCAGTACCCAG | 3 | CAGGUGAACAGCUGCCAGUACCCag | 82 | CUGGGUACUGGCAGCUGUUCACCUGGG | 161 |
| DsiKKC-04 | GGGTCAACGGGGAAAAGAAATTATT | 4 | GGGUCAACGGGGAAAAGAAAUUAtt | 83 | AAUAAUUUCUUUUCCCCGUUGACCCAU | 162 |
| DsiKKC-05 | GGTCAACGGGGAAAAGAAATTATTC | 5 | GGUCAACGGGGAAAAGAAAUUAUtc | 84 | GAAUAAUUUCUUUUCCCCGUUGACCCA | 163 |
| DsiKKC-06 | ACGGGGAAAAGAAATTATTCTGCAT | 6 | ACGGGGAAAAGAAAUUAUUCUGCat | 85 | AUGCAGAAUAAUUUCUUUUCCCCGUUG | 164 |
| DsiKKC-07 | GTCCCAGCCAGGACGGAGATAACAC | 7 | GUCCCAGCCAGGACGGAGAUAACac | 86 | GUGUUAUCUCCGUCCUGGCUGGGACCA | 165 |
| DsiKKC-08 | GAGATAACACCATCTTCAAGGCCTG | 8 | GAGAUAACACCAUCUUCAAGGCCtg | 87 | CAGGCCUUGAAGAUGGUGUUAUCUCCG | 166 |
| DsiKKC-09 | GGGCCAAGGAGACAGGGAAATACAC | 9 | GGGCCAAGGAGACAGGGAAAUACac | 88 | GUGUAUUUCCCUGUCUCCUUGGCCCAG | 167 |
| DsiKKC-10 | GGCCAAGGAGACAGGGAAATACACC | 10 | GGCCAAGGAGACAGGGAAAUACAcc | 89 | GGUGUGUUUCCCUGUCUCCUUGGCCCA | 168 |
| DsiKKC-11 | CACCGAAGGCGTGGATGAAGCCGAT | 11 | CACCGAAGGCGUGGAUGAAGCCGat | 90 | AUCGGCUUCAUCCACGCCUUCGGUGUA | 169 |
| DsiKKC-12 | CCGATCCGGCCAAGTGGAAGGCCAA | 12 | CCGAUCCGGCCAAGUGGAAGGCCaa | 91 | UUGGCCUUCCACUUGGCCGGAUCGGCU | 170 |
| DsiKKC-13 | CCGGCCAAGTGGAAGGCCAACCTGC | 13 | CCGGCCAAGUGGAAGGCCAACCUgc | 92 | GCAGGUUGGCCUUCCACUUGGCCGGAU | 171 |
| DsiKKC-14 | CGGCCAAGTGGAAGGCCAACCTGCG | 14 | CGGCCAAGUGGAAGGCCAACCUGcg | 93 | CGCAGGUUGGCCUUCCACUUGGCCGGA | 172 |

(continued)

| double stranded nucleic acid | target IRF5 cDNA. sequence | SEQ ID NO: | sense strand (5' - 3') | SEQ ID NO: | antisense strand (5' - 3') | SEQ ID NO: |
|---|---|---|---|---|---|---|
| DsiKKC-15 | TGCGCTGTGCCCTTAACAAGAGCCG | 15 | UGCGCUGUGCCCUUAACAAGAGCcg | 94 | CGGCUCUUGUUAAGGGC ACAGCGCAGG | 173 |
| DsiKKC-16 | GGGACTTCCGCCTCATCTACGACGG | 16 | GGGACUUCCGCCUCAUCUACGACgg | 95 | CCGUCGUAGAUGAGGCG GAAGUCCCGG | 174 |
| DsiKKC-17 | CAATGGCCCTGCTCCCACAGACTCC | 17 | CAAUGGCCCUGCUCCCACAGACUcc | 96 | GGAGUCUGUGGGAGCAG GGCCAUUGGA | 175 |
| DsiKKC-18 | CCCTGAGGATTACTCTTTTGGTGCA | 18 | CCCUGAGGAUUACUCUUUUGGUGca | 97 | UGCACCAAAAGAGUAAUC CUCAGGGGG | 176 |
| DsiKKC-19 | GGATTACTCTTTTGGTGCAGGAGAG | 19 | GGAUUACUCUUUUGGUGCAGGAGag | 98 | CUCUCCUGCACCAAAAGA GUAAUCCUC | 177 |
| DsiKKC-20 | GAGAGGAGGAGGAAGAAGAGGAAGA | 20 | GAGAGGAGGAGGAAGAAGAGGAAga | 99 | UCUUCCUCUUCUUCCUCC UCCUCUCCU | 178 |
| DsiKKC-21 | GGAGGAGGAAGAAGAGGAAGAGCTG | 21 | GGAGGAGGAAGAAGAGGAAGAGCtg | 100 | CAGCUCUUCCUCUUCUUC CUCCUCCUC | 179 |
| DsiKKC-22 | GCCTGAGCCTCACAGAGGATGTCAA | 22 | GCCUGAGCCUCACAGAGGAUGUCaa | 101 | UUGACAUCCUCUGUGAGG CUCAGGCUU | 180 |
| DsiKKC-23 | GCCTCACAGAGGATGTCAAGTGGCC | 23 | GCCUCACAGAGGAUGUCAAGUGGcc | 102 | GGCCACUUGACAUCCUCU GUGAGGCUC | 181 |
| DsiKKC-24 | CTGGCTTCAGGGAGCTTCTCTCTGA | 24 | CUGGCUUCAGGGAGCUUCUCUCUga | 103 | UCAGAGAGAAGCUCCCUG AAGCCAGCA | 182 |
| DsiKKC-25 | GCGAACAGCTCCTGCCAGACCTGCT | 25 | GCGAACAGCUCCUGCCAGACCUGct | 104 | AGCAGGUCUGGCAGGAG CUGUUCGCCU | 183 |
| DsiKKC-26 | CTCTGACCGACCTGGAGATCAAGTT | 26 | CUCUGACCGACCUGGAGAUCAAGtt | 105 | AACUUGAUCUCCAGGUCG GUCAGAGGC | 184 |
| DsiKKC-27 | TGACCGACCTGGAGATCAAGTTTCA | 27 | UGACCGACCUGGAGAUCAAGUUUca | 106 | UGAAACUUGAUCUCCAGG UCGGUCAGA | 185 |
| DsiKKC-28 | CGACCTGGAGATCAAGTTTCAGTAC | 28 | CGACCUGGAGAUCAAGUUUCAGUac | 107 | GUACUGAAACUUGAUCUC CAGGUCGGU | 186 |

(continued)

| double stranded nucleic acid | target IRF5 cDNA. sequence | SEQ ID NO: | sense strand (5' - 3') | SEQ ID NO: | antisense strand (5' - 3') | SEQ ID NO: |
|---|---|---|---|---|---|---|
| DsiKKC-29 | ACCTGGAGATCAAGTTTCAGTACCG | 29 | ACCUGGAGAUCAAGUUUCAGUACcg | 108 | CGGUACUGAAACUUGAUC UCCAGGUCG | 187 |
| DsiKKC-30 | GGGCCCTCACCATCAGCAACCCCCA | 30 | GGGCCCUCACCAUCAGCAACCCCca | 109 | UGGGGGUUGCUGAUGGU GAGGGCCCGG | 188 |
| DsiKKC-31 | CCCAGGAGCAGGTGGAACTCTTCGG | 31 | CCCAGGAGCAGGUGGAACUCUUCgg | 110 | CCGAAGAGUUCCACCUGC UCCUGGGUG | 189 |
| DsiKKC-32 | GGAACTCTTCGGCCCCATAAGCCTG | 32 | GGAACUCUUCGGCCCCAUAAGCCtg | 111 | CAGGCUUAUGGGGCCGA AGAGUUCCAC | 190 |
| DsiKKC-33 | AGCAGCGCTTCTACACGAACCAGCT | 33 | AGCAGCGCUUCUACACGAACCAGct | 112 | AGCUGGUUCGUGUAGAA GCGCUGCUUG | 191 |
| DsiKKC-34 | ACACGAACCAGCTGCTGGATGTCCT | 34 | ACACGAACCAGCUGCUGGAUGUCct | 113 | AGGACAUCCAGCAGCUGG UUCGUGUAG | 192 |
| DsiKKC-35 | CGAACCAGCTGCTGGATGTCCTGGA | 35 | CGAACCAGCUGCUGGAUGUCCUGga | 114 | UCCAGGACAUCCAGCAGC UGGUUCGUG | 193 |
| DsiKKC-36 | CGGGCTCATCCTCCAGCTACAGGGC | 36 | CGGGCUCAUCCUCCAGCUACAGGgc | 115 | GCCCUGUAGCUGGAGGA UGAGCCCGCG | 194 |
| DsiKKC-37 | GGCTCATCCTCCAGCTACAGGGCCA | 37 | GGCUCAUCCUCCAGCUACAGGGCca | 116 | UGGCCCUGUAGCUGGAG GAUGAGCCCG | 195 |
| DsiKKC-38 | CTGTGTCAGTGCAAGGTGTTCTGGA | 38 | CUGUGUCAGUGCAAGGUGUUCUGga | 117 | UCCAGAACACCUUGCACU GACACAGGC | 196 |
| DsiKKC-39 | CCATCCAGCGGGAGGTCAAGACCAA | 39 | CCAUCCAGCGGGAGGUCAAGACCaa | 118 | UUGGUCUUGACCUCCCG CUGGAUGGGG | 197 |
| DsiKKC-40 | AGCGGGAGGTCAAGACCAAGCTTTT | 40 | AGCGGGAGGUCAAGACCAAGCUUtt | 119 | AAAAGCUUGGUCUUGACC UCCCGCUGG | 198 |

[Table 1-2]

| double stranded nucleic acid | target IRF5 cDNA sequence | SEQ ID NO: | sense strand (5' - 3') | SEQ ID NO: | antisense strand (5' - 3') | SEQ ID NO: |
|---|---|---|---|---|---|---|
| DsiKKC-41 | GGAGGTCAAGACCAAGCTTTTCAGC | 41 | GGAGGUCAAGACCAAGCUUUUCAgc | 120 | GCUGAAAAGCUUGGUCUUGACCUCCCG | 199 |
| DsiKKC-42 | GCCTGGAGCATTTTCTCAATGAGCT | 42 | GCCUGGAGCAUUUUCUCAAUGAGct | 121 | AGCUCAUUGAGAAAAUGCUCCAGGCUG | 200 |
| DsiKKC-43 | TCAATGAGCTCATCCTGTTCCAAAA | 43 | UCAAUGAGCUCAUCCUGUUCCAAaa | 122 | UUUUGGAACAGGAUGAGCUCAUUGAGA | 201 |
| DsiKKC-44 | AGCTCATCCTGTTCCAAAAGGGCCA | 44 | AGCUCAUCCUGUUCCAAAAGGGCca | 123 | UGGCCCUUUUGGAACAGGAUGAGCUCA | 202 |
| DsiKKC-45 | CACCCTTCGAGATCTTCTTCTGCTT | 45 | CACCCUUCGAGAUCUUCUUCUGCtt | 124 | AAGCAGAAGAAGAUCUCGAAGGGUGGU | 203 |
| DsiKKC-46 | GCAAACCCCGAGAGAAGAAGCTCAT | 46 | GCAAACCCCGAGAGAAGAAGCUCat | 125 | AUGAGCUUCUUCUCUCGGGGUUUGCGG | 204 |
| DsiKKC-47 | CGAGAGAAGAAGCTCATTACTGTAC | 47 | CGAGAGAAGAAGCUCAUUACUGUac | 126 | GUACAGUAAUGAGCUUCUUCUCUCGGG | 205 |
| DsiKKC-48 | GAAGAAGCTCATTACTGTACAGGTG | 48 | GAAGAAGCUCAUUACUGUACAGGtg | 127 | CACCUGUACAGUAAUGAGCUUCUUCUC | 206 |
| DsiKKC-49 | CAGCTCGACTGCTGCTGGAGATGTT | 49 | CAGCUCGACUGCUGCUGGAGAUGtt | 128 | AACAUCUCCAGCAGCAGUCGAGCUGCU | 207 |
| DsiKKC-50 | GAGCTATCTTGGTCAGCTGATAGTA | 50 | GAGCUAUCUUGGUCAGCUGAUAGta | 129 | UACUAUCAGCUGACCAAGAUAGCUCCC | 208 |
| DsiKKC-51 | CTATCTTGGTCAGCTGATAGTATCC | 51 | CUAUCUUGGUCAGCUGAUAGUAUcc | 130 | GGAUACUAUCAGCUGACCAAGAUAGCU | 209 |
| DsiKKC-52 | AGCTGATAGTATCCGGCTACAGATC | 52 | AGCUGAUAGUAUCCGGCUACAGAtc | 131 | GAUCUGUAGCCGGAUACUAUCAGCUGA | 210 |
| DsiKKC-53 | CTGATAGTATCCGGCTACAGATCTC | 53 | CUGAUAGUAUCCGGCUACAGAUCtc | 132 | GAGAUCUGUAGCCGGAUACUAUCAGCU | 211 |
| DsiKKC-54 | GATAGTATCCGGCTACAGATCTCAA | 54 | GAUAGUAUCCGGCUACAGAUCUCaa | 133 | UUGAGAUCUGUAGCCGGAUACUAUCAG | 212 |
| DsiKKC-55 | GCTACAGATCTCAAACCCAGACCTC | 55 | GCUACAGAUCUCAAACCCAGACCtc | 134 | GAGGUCUGGGUUUGAGAUCUGUAGCCG | 213 |
| DsiKKC-56 | CGCATGGTGGAGCAATTCAAGGAGC | 56 | CGCAUGGUGGAGCAAUUCAAGGAgc | 135 | GCUCCUUGAAUUGCUCCACCAUGCGGU | 214 |
| DsiKKC-57 | GCATGGTGGAGCAATTCAAGGAGCT | 57 | GCAUGGUGGAGCAAUUCAAGGAGct | 136 | AGCUCCUUGAAUUGCUCCACCAUGCGG | 215 |
| DsiKKC-58 | CAAGGAGCTCCATCACATCTGGCAG | 58 | CAAGGAGCUCCAUCACAUCUGGCag | 137 | CUGCCAGAUGUGAUGGAGCUCCUUGAA | 216 |
| DsiKKC-59 | GCACCCAGCTGGCATGCAATAACAA | 59 | GCACCCAGCUGGCAUGCAAUAACaa | 138 | UUGUUAUUGCAUGCCAGCUGGGUGCAU | 217 |
| DsiKKC-60 | CCCAGCTGGCATGCAATAACAAGGC | 60 | CCCAGCUGGCAUGCAAUAACAAGgc | 139 | GCCUUGUUAUUGCAUGCCAGCUGGGUG | 218 |
| DsiKKC-61 | CAGCTGGCATGCAATAACAAGGCTG | 61 | CAGCUGGCAUGCAAUAACAAGGCtg | 140 | CAGCCUUGUUAUUGCAUGCCAGCUGGG | 219 |
| DsiKKC-62 | AGCTGGCATGCAATAACAAGGCTGC | 62 | AGCUGGCAUGCAAUAACAAGGCUgc | 141 | GCAGCCUUGUUAUUGCAUGCCAGCUGG | 220 |
| DsiKKC-63 | GACTGATGTGGAGATGTGACAGCCC | 63 | GACUGAUGUGGAGAUGUGACAGCcc | 142 | GGGCUGUCACAUCUCCACAUCAGUCCG | 221 |
| DsiKKC-64 | CAGGGTCCTACCTCTGGGTTTCCTG | 64 | CAGGGUCCUACCUCUGGGUUUCCtg | 143 | CAGGAAACCCAGAGGUAGGACCCUGCA | 222 |

(continued)

| double stranded nucleic acid | target IRF5 cDNA sequence | SEQ ID NO: | sense strand (5' - 3') | SEQ ID NO: | antisense strand (5' - 3') | SEQ ID NO: |
|---|---|---|---|---|---|---|
| DsiKKC-65 | GGGTTTCCTGGAAGTGGATTTGGGC | 65 | GGGUUUCCUGGAAGUGGAUUUGGgc | 144 | GCCCAAAUCCACUUCCAGGAAACCCAG | 223 |
| DsiKKC-66 | GGAAGTGGATTTGGGCCAAGAAGGA | 66 | GGAAGUGGAUUUGGGCCAAGAAGga | 145 | UCCUUCUUGGCCCAAAUCCACUUCCAG | 224 |
| DsiKKC-67 | CCATGAGCAGGGAAAGAACTCTCCC | 67 | CCAUGAGCAGGGAAAGAACUCUCcc | 146 | GGGAGAGUUCUUUCCCUGCUCAUGGCU | 225 |
| DsiKKC-68 | CCTGGGGCCTAGCTGTATAGGAGGA | 68 | CCUGGGGCCUAGCUGUAUAGGAGga | 147 | UCCUCCUAUACAGCUAGGCCCCAGGGU | 226 |
| DsiKKC-69 | CATTTCCTCTGGCAACAAAAGCCAG | 69 | CAUUUCCUCUGGCAACAAAAGCCag | 148 | CUGGCUUUUGUUGCCAGAGGAAAUGGG | 227 |
| DsiKKC-70 | CTCACTTCCTCATCTCCCTGTCCTC | 70 | CUCACUUCCUCAUCUCCCUGUCCtc | 149 | GAGGACAGGGAGAUGAGGAAGUGAGUC | 228 |
| DsiKKC-71 | CCCTGTCCTCTGAGATAATATGAGT | 71 | CCCUGUCCUCUGAGAUAAUAUGAgt | 150 | ACUCAUAUUAUCUCAGAGGACAGGGAG | 229 |
| DsiKKC-72 | GCACTTAGGTATCATATCAGATGCT | 72 | GCACUUAGGUAUCAUAUCAGAUGct | 151 | AGCAUCUGAUAUGAUACCUAAGUGCUC | 230 |
| DsiKKC-73 | ATATCAGATGCTCAAGGCTGGCAGC | 73 | AUAUCAGAUGCUCAAGGCUGGCAgc | 152 | GCUGCCAGCCUUGAGCAUCUGAUAUGA | 231 |
| DsiKKC-74 | CCCCTTCTTGAGAGTCCAAGAACCT | 74 | CCCCUUCUUGAGAGUCCAAGAACct | 153 | AGGUUCUUGGACUCUCAAGAAGGGGU | 232 |
| DsiKKC-75 | CCTTCTTGAGAGTCCAAGAACCTGG | 75 | CCUUCUUGAGAGUCCAAGAACCUgg | 154 | CCAGGUUCUUGGACUCUCAAGAAGGGG | 233 |
| DsiKKC-76 | CCAAGAACCTGGAGCAGAAATAATT | 76 | CCAAGAACCUGGAGCAGAAAUAAtt | 155 | AAUUAUUUCUGCUCCAGGUUCUUGGAC | 234 |
| DsiKKC-77 | GAACCTGGAGCAGAAATAATTTTTA | 77 | GAACCUGGAGCAGAAAUAAUUUUta | 156 | UAAAAAUUAUUUCUGCUCCAGGUUCUU | 235 |
| DsiKKC-78 | GGAGCAGAAATAATTTTTATGTATT | 78 | GGAGCAGAAAUAAUWUUAUGUAtt | 157 | AAUACAUAAAAAUUAUUUCUGCUCCAG | 236 |
| DsiKKC-79 | GATTAATGAATGTTAAAAACAGACT | 79 | GAUUAAUGAAUGUUAAAAACAGAct | 158 | AGUCUGUUUUUAACAUUCAUUAAUCCA | 237 |

[Table 2-1]

| double stranded nucleic acid | mRNA knockdown rate |
|---|---|
| siKKC3 | 0% |
| Dharmafect only | 7% |
| Mock | 11% |
| DsiKKC-01 | 44% |
| DsiKKC-02 | 34% |
| DsiKKC-03 | 29% |
| DsiKKC-04 | 45% |
| DsiKKC-05 | 38% |
| DsiKKC-06 | 46% |
| DsiKKC-07 | 48% |
| DsiKKC-08 | 39% |
| DsiKKC-09 | 47% |
| DsiKKC-10 | 41% |
| DsiKKC-11 | 25% |
| DsiKKC-12 | 22% |
| DsiKKC-13 | 35% |
| DsiKKC-14 | 27% |
| DsiKKC-15 | 36% |
| DsiKKC-16 | 28% |
| DsiKKC-17 | 19% |
| DsiKKC-18 | 52% |
| DsiKKC-19 | 44% |
| DsiKKC-20 | 49% |
| DsiKKC-21 | 55% |
| DsiKKC-22 | 55% |
| DsiKKC-23 | 24% |
| DsiKKC-24 | 37% |
| DsiKKC-25 | 54% |
| DsiKKC-26 | 54% |
| DsiKKC-27 | 52% |
| DsiKKC-28 | 62% |
| DsiKKC-29 | 66% |

[Table 2-2]

| double stranded nucleic acid | mRNA knockdown rate |
|---|---|
| DsiKKC-30 | 21% |
| DsiKKC-31 | 49% |

(continued)

| double stranded nucleic acid | mRNA knockdown rate |
|---|---|
| DsiKKC-32 | 35% |
| DsiKKC-33 | 49% |
| DsiKKC-34 | 34% |
| DsiKKC-35 | 36% |
| DsiKKC-36 | 23% |
| DsiKKC-37 | 42% |
| DsiKKC-38 | 63% |
| DsiKKC-39 | 58% |
| DsiKKC-40 | 50% |
| DsiKKC-41 | 57% |
| DsiKKC-42 | 60% |
| DsiKKC-43 | 56% |
| DsiKKC-44 | 40% |
| DsiKKC-45 | 45% |
| DsiKKC-46 | 31% |
| DsiKKC-47 | 50% |
| DsiKKC-48 | 56% |
| DsiKKC-49 | 54% |
| DsiKKC-50 | 48% |
| DsiKKC-51 | 52% |
| DsiKKC-52 | 57% |
| DsiKKC-53 | 57% |
| DsiKKC-54 | 56% |
| DsiKKC-55 | 55% |
| DsiKKC-56 | 38% |
| DsiKKC-57 | 37% |
| DsiKKC-58 | 1% |
| DsiKKC-59 | 52% |
| DsiKKC-60 | 17% |
| DsiKKC-61 | 28% |

[Table 2-3]

| double stranded nucleic acid | mRNA knockdown rate |
|---|---|
| DsiKKC-62 | 17% |
| DsiKKC-63 | 40% |
| DsiKKC-64 | 43% |
| DsiKKC-65 | 29% |

(continued)

| double stranded nucleic acid | mRNA knockdown rate |
|---|---|
| DsiKKC-66 | 29% |
| DsiKKC-67 | 24% |
| DsiKKC-68 | 46% |
| DsiKKC-69 | 9% |
| DsiKKC-70 | 52% |
| DsiKKC-71 | 56% |
| DsiKKC-72 | 58% |
| DsiKKC-73 | 39% |
| DsiKKC-74 | 43% |
| DsiKKC-75 | 39% |
| DsiKKC-76 | 57% |
| DsiKKC-77 | 59% |
| DsiKKC-78 | 56% |
| DsiKKC-79 | 27% |

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the gist and scope of the appended "CLAIMS."

The contents disclosed in any publication cited herein, including patents and patent applications, are hereby incorporated in their entireties by reference, to the extent that they have been disclosed herein.

This application is based on a US provisional application 61/952,426 filed in USA (filing date: March 13, 2014), the contents of which are incorporated in full herein.

[Industrial Applicability]

The present invention provides a nucleic acid having activity to suppress expression of IRF5, a pharmaceutical composition comprising the nucleic acid as an active ingredient, and the like. The nucleic acid and pharmaceutical composition of the present invention suppress expression of IRF5, and are useful for the treatment or prophylaxis of autoimmune diseases such as systemic lupus erythematosus, rheumatoid arthritis and the like.

## Claims

1. A double-stranded nucleic acid that decreases expression of irf5 gene, which consists of a sense strand and an antisense strand, and comprises a double-stranded region of at least 11 base pairs, wherein an oligonucleotide chain having a chain length of at least 17 nucleotides and 30 nucleotides at most in the aforementioned antisense strand is complementary to a target IRF5 mRNA sequence selected from the group consisting of SEQ ID NOs: 1 - 79.

2. The double-stranded nucleic acid according to claim 1, wherein the aforementioned double-stranded region is composed of 17 - 27 base pairs, and the 2nd nucleotide from the 5'-terminus of the aforementioned antisense strand complementary to the target IRF5 mRNA sequence selected from the group consisting of SEQ ID NOs: 1 - 79 is complement to the 2nd deoxyribonucleotide from the 3'-terminus of the target IRF5 mRNA sequence.

3. The double-stranded nucleic acid according to claim 1, wherein the 1st and 2nd nucleotides from the 3'-terminus of an oligonucleotide chain of the aforementioned sense strand are deoxyribonucleotides.

4. The double-stranded nucleic acid according to claim 1, wherein the 3'-terminus of the aforementioned sense strand and the 5'-terminus of the aforementioned antisense strand form a blunt end.

5. The double-stranded nucleic acid according to claim 1, wherein the aforementioned sense strand is 25 nucleotides in length and the aforementioned antisense strand is 27 nucleotides in length.

6. The double-stranded nucleic acid according to claim 1, wherein the aforementioned antisense strand comprises a sequence selected from the group consisting of SEQ ID NOs: 159 - 237.

7. The double-stranded nucleic acid according to claim 1, wherein the aforementioned sense strand comprises a sequence selected from the group consisting of SEQ ID NOs: 80 - 158.

8. The double-stranded nucleic acid according to claim 1, comprising one pair of the sense strand/antisense strand sequences, which is selected from the group consisting of SEQ ID NO: 83/SEQ ID NO: 162, SEQ ID NO: 85/SEQ ID NO: 164, SEQ ID NO: 86/SEQ ID NO: 165, SEQ ID NO: 88/SEQ ID NO: 167, SEQ ID NO: 97/SEQ ID NO: 176, SEQ ID NO: 99/SEQ ID NO: 178, SEQ ID NO: 100/SEQ ID NO: 179, SEQ ID NO: 101/SEQ ID NO: 180, SEQ ID NO: 104/SEQ ID NO: 183, SEQ ID NO: 105/SEQ ID NO: 184, SEQ ID NO: 106/SEQ ID NO: 185, SEQ ID NO: 107/SEQ ID NO: 186, SEQ ID NO: 108/SEQ ID NO: 187, SEQ ID NO: 110/SEQ ID NO: 189, SEQ ID NO: 112/SEQ ID NO: 191, SEQ ID NO: 117/SEQ ID NO: 196, SEQ ID NO: 118/SEQ ID NO: 197, SEQ ID NO: 119/SEQ ID NO: 198, SEQ ID NO: 120/SEQ ID NO: 199, SEQ ID NO: 121/SEQ ID NO: 200, SEQ ID NO: 124/SEQ ID NO: 203, SEQ ID NO: 126/SEQ ID NO: 205, SEQ ID NO: 127/SEQ ID NO: 206, SEQ ID NO: 128/SEQ ID NO: 207, SEQ ID NO: 129/SEQ ID NO: 208, SEQ ID NO: 130/SEQ ID NO: 209, SEQ ID NO: 131/SEQ ID NO: 210, SEQ ID NO: 132/SEQ ID NO: 211, SEQ ID NO: 133/SEQ ID NO: 212, SEQ ID NO: 134/SEQ ID NO: 213, SEQ ID NO: 138/SEQ ID NO: 217, SEQ ID NO: 147/SEQ ID NO: 226, SEQ ID NO: 149/SEQ ID NO: 228, SEQ ID NO: 150/SEQ ID NO: 229, SEQ ID NO: 151/SEQ ID NO: 230, SEQ ID NO: 155/SEQ ID NO: 234, SEQ ID NO: 156/SEQ ID NO: 235, and SEQ ID NO: 157/SEQ ID NO: 236.

9. A pharmaceutical composition comprising the double stranded nucleic acid according to any one of claims 1 to 8.

10. A method of treating an autoimmune disease, comprising a step of administering a therapeutically effective amount of the double stranded nucleic acid according to any one of claims 1 to 8 or the pharmaceutical composition according to claim 9 to a human in need of the treatment.

11. The method according to claim 10, wherein the autoimmune disease is systemic lupus erythematosus and/or rheumatoid arthritis.

12. The pharmaceutical composition according to claim 9, which is for the treatment of an autoimmune disease.

13. The pharmaceutical composition according to claim 12, wherein the autoimmune disease is systemic lupus erythematosus and/or rheumatoid arthritis.

14. The double stranded nucleic acid according to any of claims 1 to 8, which is for use for the treatment of an autoimmune disease.

**15.** The double stranded nucleic acid according to claim 14, wherein the autoimmune disease is systemic lupus erythematosus and/or rheumatoid arthritis.

**16.** Use of the double stranded nucleic acid according to any of claims 1 to 8 in the production of a therapeutic agent for an autoimmune disease.

**17.** The use according to claim 16, wherein the autoimmune disease is systemic lupus erythematosus and/or rheumatoid arthritis.

## INTERNATIONAL SEARCH REPORT

International application No.
PCT/JP2015/057353

A. CLASSIFICATION OF SUBJECT MATTER
*C12N15/113*(2010.01)i

According to International Patent Classification (IPC) or to both national classification and IPC

B. FIELDS SEARCHED

Minimum documentation searched (classification system followed by classification symbols)
C12N15/113

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/MEDLINE/EMBASE/BIOSIS(STN), JSTPlus/JMEDPlus/JST7580(JDreamIII), GenBank/EMBL/DDBJ/GeneSeq, PubMed

C. DOCUMENTS CONSIDERED TO BE RELEVANT

| Category* | Citation of document, with indication, where appropriate, of the relevant passages | Relevant to claim No. |
|---|---|---|
| X/Y | WO 2005/116204 A1 (RNAi Co., Ltd.), 08 December 2005 (08.12.2005), pages 761, 762, 337; claim 18 & US 2008/0113351 A1 & US 2011/0054005 A1 & EP 1752536 A1 | 1,2,9/1-9, 12-17 |
| Y | SCHOENEMEYER, A., et al., The interferon regulatory factor, IRF5, is a central mediator of toll-like receptor 7 signaling, 2005, The Journal of Biological Chemistry, Vol.280, No.17, p.17005-17012, page 17006, right column, 4th paragraph | 1-9,12-17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 June 2015 (05.06.15) | 16 June 2015 (16.06.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/057353

C (Continuation). DOCUMENTS CONSIDERED TO BE RELEVANT

| Category* | Citation of document, with indication, where appropriate, of the relevant passages | Relevant to claim No. |
|---|---|---|
| Y | HU, G., et al., Signaling through IFN regulatory factor-5 sensitizes p53-deficient tumors to DNA damage-induced apoptosis and cell death, 2005, Cancer Research, Vol.65, No.16, p.7403-7412, page 7404, left column, 5th paragraph | 1-9,12-17 |
| Y | COURTIES, G., et al., In vivo silencing of the transcription factor IRF5 reprograms the macrophage phenotype and improves infarct healing, Epub 2013 DEC., Journal of the American College of Cardiology, Vol.63, No.15, p.1556-1566, page 1559, right column | 1-9,12-17 |
| Y | Yoshifumi TADA et al., "Interferon Seigyo Inshi 5(IRF5) no Lupus Mouse no Byotai Hatsugen ni Okeru Sayo to sono Mechanism", 2011, Japanese journal of clinical immunology Dai 39 Kai The Japan Society for Clinical Immunology Sokai Shorokushu Go, vol.34, no.4, page 284, PW-7, entire text | 1-9,12-17 |
| Y | Yoshifumi TADA et al., "Collagen Yudo Kansetsuen ni Okeru Interferon Chosetsu Inshi 5(IRF5) no Sayo", 2013, Japanese journal of clinical immunology Dai 41 Kai The Japan Society for Clinical Immunology Sokai Shorokushu Go, vol.36, no.5, page 381, P3-04, entire text | 1-9,12-17 |
| A | JP 2013-112674 A (Kowa Co., Ltd.), 10 June 2013 (10.06.2013), entire text (Family: none) | 1-9,12-17 |

Form PCT/ISA/210 (continuation of second sheet) (July 2009)

**INTERNATIONAL SEARCH REPORT**

International application No.
PCT/JP2015/057353

**Box No. II Observations where certain claims were found unsearchable (Continuation of item 2 of first sheet)**

This international search report has not been established in respect of certain claims under Article 17(2)(a) for the following reasons:

1. [X] Claims Nos.: 10,11
because they relate to subject matter not required to be searched by this Authority, namely:
Claims 10 and 11 pertain to methods for therapy of the human body and thus relate to a subject matter on which this International Searching Authority is not required to carry out a search under the provisions of PCT Article 17(2)(a)(i) and PCT Rule 39.1(iv).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012093258 A
- WO 2012005898 A
- WO 2005018534 A
- WO 2005089287 A
- US 20120040459 A
- WO 2006080118 A
- US 61952426 B


### Non-patent literature cited in the description


- *Nat. Immun.,* 2011, vol. 12 (3), 231-238
- *Genes. Immun.,* 2007, vol. 8, 445-455
- *Nat. Genet.,* 2006, vol. 38, 550-555
- *Proc. Natl. Acad. Sci. U.S.A.,* 2007, vol. 104, 6758-6763
- *Arthritis. Rheum.,* 2007, vol. 56, 1234-1241
- *Arthritis. Rheum.,* 2008, vol. 58, 826-834
- *Arthritis. Rheum.,* 2009, vol. 60, 1845-1850
- *J. Biol. Chem.,* 2005, vol. 280 (17), 17005-17012
- *Cancer Res.,* 2005, vol. 65 (16), 7403-7412
- *Blood,* 2010, vol. 115 (22), 4421-4430
- *Nat. Immunol.,* 2011, vol. 12 (3), 231-238
- *J. Virol.,* 2005, vol. 79 (18), 11671-11676
- *Nucleic Acid Research,* 2004, vol. 32, e175
- *Acc. Chem. Res.,* 1999, vol. 32, 624
- *J. Am. Chem. Soc.,* 2001, vol. 123, 4653
- *J. Am. Chem. Soc.,* 2000, vol. 122, 6900
- *Nucleic Acid Research,* 2007, vol. 35, 3287